(19) Europäisches Patentamt
European Patent Office
Office européen des brevets

(11) Veröffentlichungsnummer **0 010 520**
**B1**

(12) **EUROPÄISCHE PATENTSCHRIFT**

(45) Veroffentlichungstag der Patentschrift: 16.12.81

(21) Anmeldenummer: 79810122.6

(22) Anmeldetag: 12.10.79

(51) Int Cl³: **C 07 C 131/00**, C 07 C 109/12, C 07 C 133/04, C 07 C 119/08, C 07 D 307/52 // C07C87/14, C08G69/26, C08G59/50

(54) **Substituierte 11-amino-undeca-4,8-dienal- und 11-Amino-undecanal-derivate sowie Verfahren zu ihrer Herstellung.**

(30) Priorität 18.10.78 CH 10769/78

(43) Veroffentlichungstag der Anmeldung: 30.04.80 Patentblatt 80/9

(45) Bekanntmachung des Hinweises auf die Patenterteilung: 16.12.81 Patentblatt 81/50

(84) Benannte Vertragsstaaten
CH DE FR GB IT NL

(56) Entgegenhaltungen:
DE-A-2 330 087
DE-A1-2 831 385
GB-A-1 251 520
GB-A-1 410 005
US-A-3 849 469

(73) Patentinhaber: CIBA-GEIGY AG, Patentabteilung Postfach, CH-4002 Basel (CH)

(72) Erfinder: Reinehr, Dieter, Dr., Wolfsheule 10, D-7842 Kandern (DE)
Erfinder: Pfeifer, Josef, Dr., Brunnmattstrasse 32, CH-4106 Therwil (CH)

EP 0 010 520 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Substituierte 11-Amino-undeca-4,8-dienal- und 11-Amino-undecanalderivate sowie Verfahren zu ihrer Herstellung

Die vorliegende Erfindung betrifft neue substituierte 11-Amino-undeca-4,8-dienal- und 11-Amino-undecanalaldehydderivate und Verfahren zu deren Herstellung. Die neuen 11-Amino-undeca-4,8-dienal- und 11-Amino-undecanal-aldehydderivate stellen wertvolle Zwischenprodukte zur Herstellung von substituierten 1,11-Diaminoundecanen dar.

Es ist bekannt, daß sich gegebenenfalls substituierte Alkylendiamine zur Herstellung von transparenten Polyamiden eignen. So werden z. B. in der deutschen Offenlegungsschrift 1 720 513 generisch kochbeständige, transparente Polyamide aus aromatischen Dicarbonsäuren und gegebenenfalls alkylsubstituierten Alkylendiaminen mit 1—10 C-Atomen in der Kette, die an mindestens einem der beiden endständigen C-Atome durch eine Alkylgruppe mit 1—4 C-Atomen substituiert sind, beschrieben. Die konkrete Offenbarung beschränkt sich jedoch auf transparente Polyamide aus aromatischen Dicarbonsäuren und Alkylendiaminen der vorerwähnten Art mit höchstens 7 C-Atomen in der Kette. In den britischen Patentschriften 905 475 und 919 096 sind weitere transparente Polyamide aus Terephthalsäure, Isophthalsäure oder Gemischen davon und Hexamethylendiaminen mit mindestens drei durch Alkylsubstitution eingeführten C-Atomen in einer oder mehreren Seitenketten, wie 2,2,4- und 2,4,4-Trimethylhexamethylendiamin, 2-Methyl-4-äthylhexymethylendiamin und 2-Äthyl-4-methylhexamethylendiamin oder Isomerengemischen solcher Hexamethylendiamine beschrieben. Alkylendiamine eignen sich auch als Co-Kondensationskomponente für die Herstellung von transparenten Polyamiden aus 4,4'-Diaminodicyclohexylalkanen und aromatischen Dicarbonsäuren und gegebenenfalls weiteren Co-Kondensationskomponenten, wie Aminocarbonsäuren oder deren Lactame und aliphatische Dicarbonsäuren. Derartige Polyamide sind z. B. in der US-Patentschrift 3 597 400 und der deutschen Offenlegungsschrift 2 642 244 beschrieben. Diese vorbekannten Polyamide und Copolyamide lassen aber hinsichtlich der Wasseraufnahme, der Hydrolysebeständigkeit, der Wärmeformbeständigkeit und/oder der Dimensionsstabilität unter Feuchtigkeitseinwirkung zu wünschen übrig, wodurch auch die mechanischen und elektrischen Eigenschaften dieser Polyamide beeinträchtigt werden. Die genannten Polyamide sind ferner zum Teil nur schwer thermoplastisch verarbeitbar oder aber spröde Produkte.

Es wurden nun neue substituierte 11-Amino-undeca-4,8-dienal- und 11-Amino-undecanal-aldehydderivate gefunden, die sich in substituierte 1,11-Aminoundecane überführen lassen, die sich ihrerseits zur Herstellung von transparenten Polyamiden eignen, die von den oben erwähnten Nachteilen frei sind.

Die neuen substituierten 11-Amino-undeca-4,8-dienal und 11-Amino-undecanal-aldehydderivate entsprechen den Formeln Ia

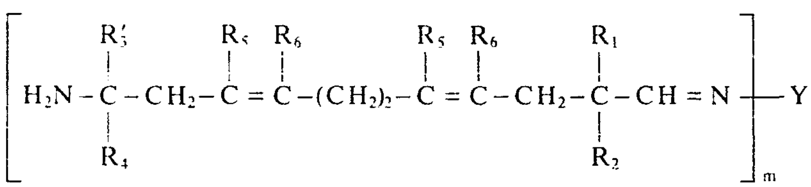

(Ia)

oder Ib

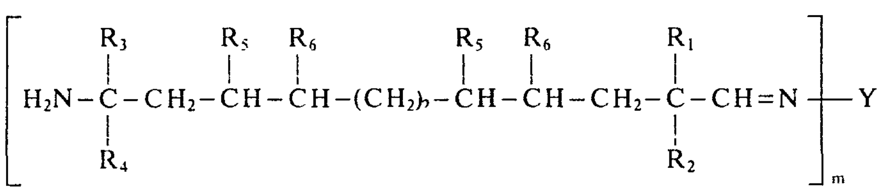

(Ib)

worin

m die Zahl 1 oder 2, Y, bei m = 1,

$-OH$, $-NH_2$, 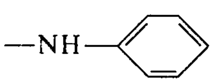, 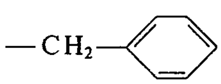 oder $-NHCONH_2$

und bei m = 2 die direkte Bindung,

$R_1$ Alkyl mit 1—12 C-Atomen,
$R_2$ Wasserstoff oder Alkyl mit 1—12 C-Atomen,

$R_3$ Alkyl mit 1–12 C-Atomen, Cycloalkyl mit 4–12 Ring-C-Atomen, Aralkyl mit 7 oder 8 C-Atomen, Aryl, das mit einer oder mehreren $C_1-C_4$ Alkylgruppen substituiert sein kann, Pyridyl, Furyl oder Thienyl,

$R_3'$ dasselbe wie $R_3$ oder, wenn $R_4$ Wasserstoff ist, auch $-CH=CH-$Alkyl oder $-C(Alkyl)=CH-$Alkyl mit je 1–4 C-Atomen in den Alkylteilen,

$R_4$ Wasserstoff, Alkyl mit 1–12 C-Atomen, Cycloalkyl mit 4–12 Ring-C-Atomen, Aralkyl mit 7 oder 8 C-Atomen oder gegebenenfalls mit einer oder mehreren $C_1-C_4$ Alkylgruppen substituiertes Aryl oder $R_1$ und $R_2$ und/oder $R_3$ und $R_4$ bzw. $R_3'$ und $R_4$ zusammen Alkylen mit 3–11 C-Atomen und

$R_5$ und $R_6$ unabhängig voneinander Wasserstoff oder Methyl bedeuten.

Durch $R_1$ bis $R_4$ dargestellte Alkylgruppen können geradkettig oder verzweigt sein. Alkylgruppen $R_1$, $R_2$ und $R_4$ weisen bevorzugt 1–5 C-Atome auf und sind geradkettig. Alkylgruppen $R_3$ oder $R_3'$ weisen mit Vorteil 1–7 C-Atome auf; besonders bevorzugt sind verzweigte Alkylgruppen $R_3$ bzw. $R_3'$ mit 1–7 C-Atomen. Beispiele von Alkylgruppen $R_1$ bis $R_4$ sind: die Methyl-, Äthyl-, n-Propyl-, Isopropyl-, n-, sek- und tert-Butyl-, n-Pentyl-, 2- oder 3-Pentyl-, n-Hexyl-, 2- oder 3-Heptyl-, n-Octyl-, n-Decyl- und n-Dodecylgruppe.

Stellt $R_3'$ eine Gruppe $-CH=CH$-Methyl oder $-C(Alkyl)=CH-$Alkyl dar, so sind die Alkylteile in diesen Gruppen bevorzugt, geradkettig und bedeuten insbesondere Methyl oder Äthyl.

Cycloalkylgruppen $R_3$, $R_3'$ und $R_4$ können unsubstituiert oder durch $C_{1-4}$-Alkylgruppen substituiert sein. Insbesondere handelt es sich dabei um durch eine Methyl- oder Äthylgruppe substituiertes Cycloalkyl.

Bevorzugt sind Cycloalkylgruppen $R_3$, $R_3'$ und $R_4$ jedoch unsubstituiert und weisen 5–8 Ring-C-Atome auf. Besonders bevorzugt sind die Cyclopentyl- und vor allem die Cyclohexylgruppe.

Als Aralkylgruppen $R_3$, $R_3'$ und $R_4$ kommen vor allem die Benzyl-, Methylbenzyl- oder Phenyläthylgruppe in Betracht. Stellt $R_3$, $R_3'$ oder $R_4$ mit Alkylgruppen substituiertes Aryl dar, so kommen Alkylgruppen mit insbesondere 1 oder 2 C-Atomen in Betracht. Arylgruppen $R_3$, $R_3'$ und $R_4$ sind bevorzugt nur durch eine Alkylgruppe substituiert. Besonders bevorzugt sind die 1- oder 2-Naphthylgruppe, durch eine Alkylgruppe mit 1–4 und besonders 1 oder 2 C-Atomen substituiertes Phenyl und ganz besonders unsubstituiertes Phenyl.

Als Pyridyl-, Furyl- oder Thienylgruppen $R_3$ bzw. $R_3'$ kommen vor allem die Pyridyl-3-, Pyridyl-4-, Furyl-2- und Thienyl-2-gruppen in Betracht.

Durch $R_1$ und $R_2$ und/oder $R_3$ und $R_4$ bzw. $R_3'$ und $R_4$ zusammen dargestellte Alkylengruppen weisen bevorzugt 4–7 C-Atome auf. Insbesondere handelt es sich dabei um die Tetramethylen- und ganz besonders die Pentamethylengruppe.

Bevorzugt sind Verbindungen der Formel Ia und Ib, worin $R_1$ Alkyl mit 1–5 C-Atomen, $R_2$ Wasserstoff oder Alkyl mit 1–5 C-Atomen oder $R_1$ und $R_2$ zusammen Alkylen mit 4–7 C-Atomen, $R_3$ Alkyl mit 1–7 C-Atomen, Cycloalkyl mit 5–8 C-Atomen oder unsubstituiertes Phenyl, $R_3'$ dasselbe wie $R_3$ oder, bei $R_4 = H$, auch $-C(C_2'H_5)=CH-CH_3$, $R_4$ Wasserstoff oder Alkyl mit 1–5 C-Atomen und $R_5$ und $R_6$ je Wasserstoff bedeuten.

Besonders bevorzugt sind Verbindungen der Formel Ia und Ib, worin m die Zahl 1, Y $-OH$, $R_1$ Alkyl mit 1–5 C-Atomen, $R_2$ Alkyl mit 1–5 C-Atomen oder Wasserstoff oder $R_1$ und $R_2$ zusammen Alkylen mit 4–7 C-Atomen, $R_3$ verzweigtes Alkyl mit 3–7 C-Atomen oder Cycloalkyl mit 5–8 C-Atomen, $R_3'$ dasselbe wie $R_3$ oder $-C(C_2H_5)=CH-CH_3$ und $R_4$, $R_5$ und $R_6$ je Wasserstoff bedeuten. Ganz besonders bevorzugt sind Verbindungen der Formel Ia und Ib, worin m die Zahl 1, Y $-OH$, $R_1$ und $R_2$ je Methyl oder Äthyl oder zusammen $C_{4-7}$-Alkylen, besonders Pentamethylen, $R_3$ und $R_3'$ Isopropyl, 3-Pentyl, $C_{5-8}$-Cycloalkyl, besonders Cyclohexyl, und $R_4$, $R_5$ und $R_6$ je Wasserstoff bedeuten.

Die Verbindungen der Formel Ia und Ib können dadurch hergestellt werden, daß man

A) eine Verbindung der Formel II

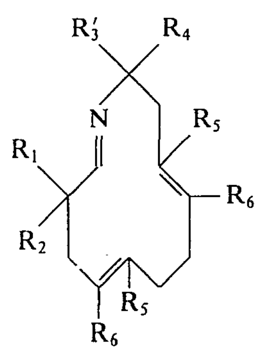

(II)

mit einer Verbindung der Formel IIIa oder IIIb

$$H_2N-Y \qquad \text{(IIIa)}$$

oder

$$[H_2NY \cdot H^{\oplus}]_n \quad X^{\ominus n}$$ (IIIb)

zu einer Verbindung der Formel Ia umsetzt, oder
B) eine Verbindung der Formel II katalytisch zu einer Verbindung der Formel IV

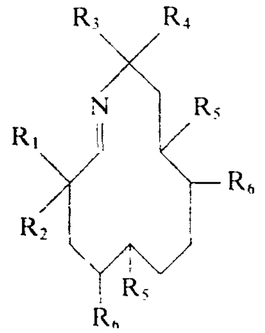

(IV)

hydriert und die Verbindung der Formel IV mit einer Verbindung der Formel IIIa oder IIIb zu einer Verbindung der Formel Ib umsetzt. In den obigen Formeln II, IIIa, IIIb und IV haben $R_1$ bis $R_6$ die unter Formel Ia bzw. Ib angegebene Bedeutung, Y hat dieselbe Bedeutung wie in den Formeln Ia und Ib, stellt aber nicht eine direkte Bindung dar, X stellt das Anion einer anorganischen, unter den Reaktionsbedingungen nicht-oxidierenden Säure dar und n bedeutet eine der Wertigkeit von X entsprechende ganze Zahl.

Verbindungen der Formel Ib können auch durch katalytische Hydrierung von Verbindungen der Formel Ia erhalten werden. Bevorzugt ist jedoch die Herstellung von Verbindungen der Formel Ib nach dem unter B) beschriebenen Verfahren.

Die Umsetzung der 1-Aza-1,5,9-cyclododecatriene der Formel II und der 1-Aza-cyclododecene der Formel IV wird zweckmäßig in wäßrigem Medium bei Temperaturen zwischen etwa 20 und 100°C vorgenommen. X stellt z. B. das Anion der Chlor- oder Bromwasserstoffsäure, der Schwefel- oder Phosphorsäure dar. Als Verbindungen der Formel IIIa oder IIIb verwendet man z. B. Phenylhydrazin, Benzylamin oder die entsprechenden Hydrochloride oder Hydrobromide, Hydroxylamin-hydrochlorid, Hydroxylaminsulfat, Hydroxylamin-hydrogensulfat, Semicarbazidhydrochlorid, Hydrazinsulfat, Hydrazinmonohydrobromid oder -chlorid. Die Verbindung der Formel IIIa mit Y = $-NH_2$ wird bevorzugt in Form des Hydrats eingesetzt.

Die Verbindungen der Formel II und IV werden zweckmäßig in im wesentlichen stöchiometrischer Menge, bezogen auf die Verbindung der Formel IIIa oder IIIb, eingesetzt. Mit Vorteil verwendet man jedoch einen leichten Überschuß an Verbindung der Formel IIIa oder IIIb. Werden die Verbindungen der Formel II bzw. IV und die Verbindungen der Formel IIIa oder IIIb mit Y = $-NH_2$ in einem Molverhältnis von etwa 1 : 1 eingesetzt, so erhält man Verbindungen der Formel Ia oder Ib mit m = 1 und Y = $-NH_2$. Bei einem Mol-Verhältnis von Verbindung der Formel II oder IV zu Verbindung der Formel IIIa oder IIIb mit Y = $-NH_2$ von mindestens 2 : 1 entstehen hingegen Verbindungen der Formel Ia oder Ib mit m = 2 und Y = direkte Bindung.

Bei den obigen Umsetzungen können intermediär Verbindungen der Formeln Va oder Vb

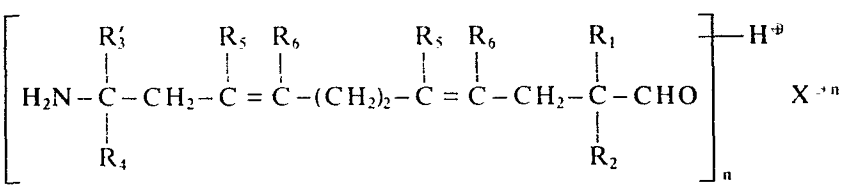

(Va)

oder

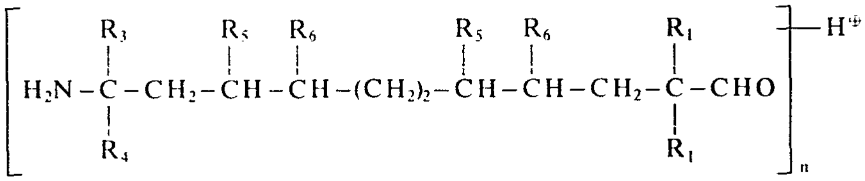

(Vb)

entstehen, wobei $R_1$ bis $R_6$, X und n die im Vorangehenden angegebene Bedeutung haben.

Im allgemeinen empfiehlt es sich, die Umsetzung unter Zusatz einer anorganischen, unter den Reaktionsbedingungen nicht-oxidierenden Säure, wie verdünnte HCl oder Schwefelsäure, durchzuführen, insbesondere bei Verwendung von Verbindungen der Formel IIIa.

Nach beendeter Umsetzung wird das Reaktionsgemisch zweckmäßig durch Zugabe einer geeigneten organischen oder anorganischen Base, wie Alkalimetall- oder Erdalkalimetallhydroxiden, -carbonaten oder -hydrogencarbonaten, tertiären Aminen, z. B. Triäthylamin oder Pyridin, neutralisiert. Bevorzugt verwendet man als Base ein Alkalimetallhydroxid, besonders Natrium- oder Kaliumhydroxid.

Die katalytische Hydrierung der 1-Aza-1,5,9-cyclododecatriene der Formel II zu 1-Aza-cyclododecenen der Formel IV wie auch die katalytische Hydrierung von 11-Aminoundeca-4,8-dienal-aldehydderivaten der Formel Ia zu 11-Amino-undecanal-aldehydderivaten der Formel Ib kann nach an sich bekannten Methoden in Gegenwart eines geeigneten inerten organischen Lösungsmittels, wie aliphatischen oder cycloaliphatischen Kohlenwasserstoffen, z. B. n-Hexan, N-Hexan, Cyclopentan oder Cyclohexan, oder cyclischen Äthern, z. B. Tetrahydrofuran, Tetrahydropyran oder Dioxan, vorgenommen werden. Bevorzugte Lösungsmittel sind Cyclohexan und Tetrahydrofuran. Als Katalysatoren verwendet man zweckmäßig Edelmetall-Katalysatoren, wie Platin-, Rhodium-, Palladium- und Ruthenium-Katalysatoren. Bevorzugt sind Rhodium/Aluminiumoxid- und Palladium/Kohle-Katalysatoren. Im allgemeinen wird die Hydrierung in geschlossenem System bei einem Druck von etwa 1–200 bar, insbesondere 1–130 bar, und bei Temperaturen zwischen etwa 0 und 150° C, vor allem zwischen etwa 25 und 100° C, vorgenommen.

Die Ausgangsprodukte der Formel IIIa und IIIb sind bekannt. Die Verbindungen der Formel II können auf analoge Weise wie in Helv. Chim. Acta, 61, Fasc. 3, 1122–1124 (1978) beschrieben durch nickelkatalysierte Mischoligomerisation von 2-Aza-1,3-butadienen der Formel VI

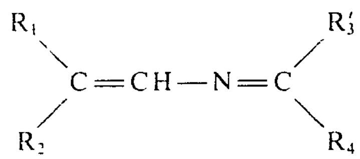

(VI)

mit Verbindungen der Formel VII

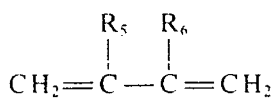

(VII)

hergestellt werden, wobei $R_1$ bis $R_6$ die unter den Formeln Ia und Ib angegebene Bedeutung haben. Geeignete Katalysatorsysteme sind z. B. in der deutschen Offenlegungsschrift 2 330 087 beschrieben. Bevorzugt sind Katalysatoren, die in situ durch Reduktion einer kohlenoxidfreien Nickelverbindung, wie Nickelstearat und vor allem Nickelacetylacetonat, mit halogenfreien Metallarylen oder Metallalkylen, z. B. Äthoxydiäthylaluminium, in Gegenwart eines Alkyl- oder Arylphosphins oder in Gegenwart eines Alkyl- oder Arylphosphits, erhalten werden.

Die obige Umsetzung wird zweckmäßig in Gegenwart eines inerten organischen Lösungsmittels, wie n-Hexan, n-Heptan, Benzol, Toluol, Diäthyläther oder Dioxan, bei Temperaturen zwischen etwa −40° C und +150° C durchgeführt.

Die 2-Aza-1,3-butadiene der Formel VI sind größtenteils bekannt oder können beispielsweise wie folgt hergestellt werden:

– durch Umsetzung von Aldehyden $R_3'-CHO$ oder Ketonen

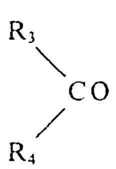

mit Alkenylaminen

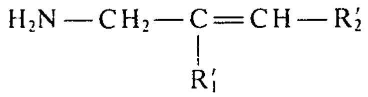

[$R_1'$ = H oder Alkyl C 1–12, $R_2'$ = H oder Alkyl C 1–11] oder Benzylamin, wobei $R_3'$ im letzteren Falle eine Gruppe $-C(Alkyl) = CH-Alkyl$ oder $-CH=C_4-Alkyl$ darstellen muß, und anschließende

Isomerisierung der erhaltenen Verbindung in Gegenwart von Katalysatoren, wie $K_2O/Al_2O_3$-Katalysatoren, Alkalimetall- oder Erdalkalimetallalkoholaten [vgl. z. B. B. A. Kazanskii et al., Zhurnal Organicheskoi Khimii, 6, No. 11, 2197—99 (1970); Izw. Akad. Nauk SSSR, Ser. Khim., No. 9, 2038—2045 (1975), Tetrahedron, 34, 833—839 (1978) und J. Org. Chem. 43, 782—784 (1978)], — durch Umsetzung von Allylamin oder Methallylamin mit Aldehyden ($R_1$) ($R_2''$) $-CH-CHO$ [$R_2''$ wie $R_2$, aber ungleich H] und anschließende Isomerisierung der erhaltenen Verbindungen ($R_1$) ($R_2''$) $-CH-CH=N-CH_2-C(R)=CH_2$ [R = H oder Methyl] in Gegenwart von Katalysatoren, wie Kalium-tert-butylat; — durch Umsetzung von Aldehyden $R_1-CH(R_2'')-CHO$ [$R_2''$ gleich wie $R_2$, aber ungleich H] mit Ammoniak (vgl. z. B. US-Patentschrift 2 319 848) und allfällige weitere Umsetzung der dabei erhaltenen Verbindungen ($R_1$) ($R_2''$)

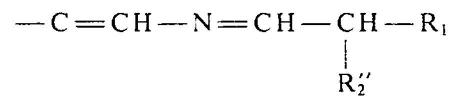

mit geeigneten Ketonen oder Aldehyden (vgl. z. B. US-Patentschrift 3 706 802).

Die Verbindungen der Formel Ia und Ib können durch katalytische Hydrierung in neue Diamine der Formel VIII

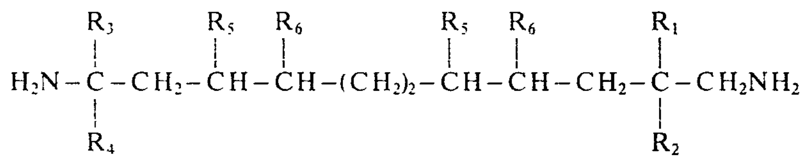

(VIII)

übergeführt werden, wobei $R_1$ bis $R_6$ die unter den Formeln Ia und Ib angegebene Bedeutung haben. Die Hydrierung wird zweckmäßig in geschlossenem System bei 1—200 bar und insbesondere 1—130 bar, und in Gegenwart eines inerten organischen Lösungsmittels, wie Methanol, Äthanol, Propanol oder Isopropanol und gegebenenfalls in Gegenwart von flüssigem Ammoniak oder Natriumhydroxid vorgenommen. Als Katalysatoren verwendet man bevorzugt Nickel-Katalysatoren, besonders Raney-Nickel.

Die Diamine der Formel VIII eignen sich beispielsweise zur Herstellung von Polykondensationsprodukten, vor allem Polyamiden oder als Härter für Epoxidharze. Transparente Polyamide, die durch Polykondensation von Diaminen der Formel I, worin $R_3$ heterocyclischer Rest, mit aromatischen Dicarbonsäuren, vor allem Terephthalsäure und/oder Isophthalsäure und gegebenenfalls weiteren langkettigen Diaminen und aliphatischen Dicarbonsäuren erhalten werden können, zeichnen sich durch hohe Glasumwandlungstemperaturen und dementsprechend hohe Wärmeformbeständigkeit, gute thermoplastische Verarbeitbarkeit, z. B. nach dem Spritzguß- oder Extrusionsverfahren, geringe Wasseraufnahme verbunden mit verminderter Abhängigkeit der mechanischen und elektrischen Eigenschaften von der Umgebungsfeuchtigkeit, verbesserte Hydrolysebeständigkeit sowie Kochwasserbeständigkeit aus.

## Beispiel 1

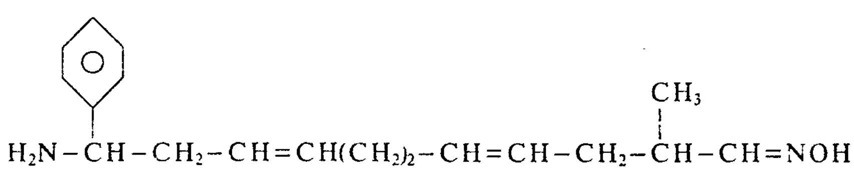

a) 7,7 g (0,03 Mol) Nickelacetylacetonat und 7,85 g (0,03 Mol) Triphenylphosphin werden unter Argon in 200 g absolutem Toluol gelöst, worauf man die Lösung bei 20—25°C mit 1,3-Butadien sättigt. Anschließend werden unter schwachem Einleiten von 1,3-Butadien langsam 10,2 g (0,09 Mol) Äthoxy-diäthylaluminium zugetropft. Es wird auf 70°C aufgeheizt, und unter starkem Einleiten von 1,3-Butadien werden innerhalb von 1 Stunde 495 g (3,41 Mol) N-Benzylidenpropenylamin so zugetropft, daß das eingeleitete Butadien gerade verbraucht wird. Es wird noch 1 Stunde unter stetigem Einleiten von 1,3-Butadien bei 70°C nachgerührt und dann auf 20—25°C abgekühlt. Zur Inaktivierung des Katalysators wird die Reaktionslösung mit 0,96 g (0,03 Mol) Schwefel versetzt und destilliert. Man erhält 750 g (2,97 Mol) 3-Methyl-12-phenyl-1-aza-1,5,9-cyclododecatrien als cis-trans-Isomerengemisch (cis : trans = 65 : 35); Sdp. 112—113° C/1 Pa; $n_D^{20}$ = 1,5505; Smp. (cis-Isomeres) = 57—58° C.

b) 502 g (1,98 Mol) 3-Methyl-12-phenyl-1-aza-1,5,9-cyclododecatrien werden innerhalb von 1,5 Stunden so zu 220 g 37%iger Salzsäure zugetropft, daß die Temperatur nicht über 80° C ansteigt. Dann wird auf Raumtemperatur (20—25°C) abgekühlt und mit 140 g (2,02 Mol) Hydroxylaminhydrochlorid versetzt. Während einer Stunde werden unter Kühlung mit einem Wasserbad ca. 185 g (4,6 Mol) festes

Natriumhydroxid zugegeben, bis der pH der wäßrigen Lösung 10—11 beträgt. Die sich abscheidende organische Phase wird abgetrennt und mit Wasser neutral gewaschen. Man erhält 567 g (1,98 Mol) 2-Methyl-11-phenyl-11-amino-undeca-4,8-dienal-oxim als hochviskose Flüssigkeit; Ausbeute 100% d. Th.

Analyse für $C_{18}H_{26}N_2O$ (Molgewicht 286,42):

| | | | | |
|---|---|---|---|---|
| berechnet | C 75,48 | H 9,15 | N 9,78 | O 5,59% |
| gefunden | C 76,00 | H 9,33 | N 9,28 | O 5,20%. |

MS-Spektrum: Molekül-Peak 286, Bruchstückmassen 269, 254, 214, 148, 121, 106.

Beispiel 2

a) 253 g (1 Mol) 3-Methyl-12-phenyl-1-aza-1,5,9-cyclododecatrien werden in 2 Liter Cyclohexan gelöst und bei 20—25°C und einem Anfangsdruck von 100 bar in Gegenwart von 40 g Rhodium/Aluminiumoxid während 4 Stunden in einem Stahlautoklav hydriert. Nach dem Abdestillieren des Lösungsmittels erhält man als Hauptfraktion 242 g (0,94 Mol) 3-Methyl-12-phenyl-1-aza-cyclododecen als cis-trans-Isomerengemisch; Sdp. 112°C/4 Pa. MS-Spektrum: Molekül-Peak 257, Bruchstückmassen 242, 172, 146, 117, 104.

b)

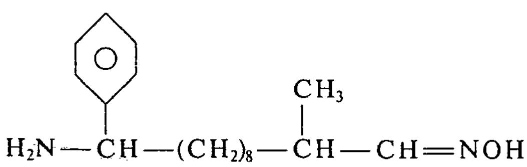

Es wird wie in Beispiel 1b) beschrieben vorgegangen, jedoch unter Verwendung von 146 g (0,568 Mol) 3-Methyl-12-phenyl-1-aza-cyclodecen, 47 g (0,2875 Mol) Hydroxylaminsulfat, 60 g (0,61 Mol) Schwefelsäure und 200 ml Wasser. Nach der Aufarbeitung wie in Beispiel 1b) beschrieben, erhält man 165 g (0,567 Mol) 2-Methyl-11-phenyl-11-amino-undecanal-oxim als hochviskose Flüssigkeit; Ausbeute 99,8% d. Th.

Analyse für $C_{18}H_{30}N_2O$ (Molgewicht 290,45):

| | | | | |
|---|---|---|---|---|
| berechnet | C 74,44 | H 10,41 | N 9,64 | O 5,51% |
| gefunden | C 74,9 | H 10,1 | N 9,1 | O 5,2% |

MS-Spektrum: Molekül-Peak 290, Bruchstückmassen 273, 259, 216, 174, 146, 106.

Beispiel 3

a) Es wird wie in Beispiel 1a) beschrieben vorgegangen, jedoch unter Verwendung von 48,5 g (0,437 Mol) N-Propyliden-(2-methylpropenylamin) [1-Äthyl-4,4-dimethyl-2-aza-1,3-butadien] und 61,0 g (1,13 Mol) 1,3-Butadien. Durch Destillation erhält man 62,0 g (0,283 Mol) 3,3-Dimethyl-12-äthyl-1-aza-1,5,9-cyclododecatrien; Sdp. 65—66°C/0,7 Pa; $n_D^{20}$ = 1,4864.

Das N-Propyliden-(2-methylpropenylamin) wurde wie folgt hergestellt: 25 g (0,233 Mol) Kalium-tert-butylat werden in einem Liter wasserfreiem Diäthyläther aufgeschlämmt. Dann tropft man innerhalb von 1 Stunde unter stetigem Rühren 921 g (8,3 Mol) Isobutyliden-allylamin so zu, daß die Temperatur des Reaktionsgemisches nicht über 20°C ansteigt. Nach beendetem Eintropfen wird noch 5 Std. bei 20—22°C weitergerührt. Dann wird die Umsetzung unterbrochen, und das Lösungsmittel wird bei einer Badtemperatur von 40°C/27 000—35 000 Pa überdestilliert. Der Rückstand wird bei einer Badtemperatur von 70°C/13 Pa in eine mit $CO_2$/Methanol gekühlte Vorlage destilliert. Nach anschließender Feindestillation erhält man 808 g (7,93 Mol) N-Propyliden-(2-methyl-propenylamin); Sdp. 122°C; $n_D^{20}$ = 1,471.

b) Es wird wie in Beispiel 1b) beschrieben vorgegangen, jedoch unter Verwendung von 219 g (1 Mol) 3,3-Dimethyl-12-äthyl-1-aza-1,5,9-cyclododecatrien, 147 g (1,5 Mol) Schwefelsäure und 82 g (0,5 Mol) Hydroxylaminsulfat. Nach der Aufarbeitung erhält man 222 g (0,879 Mol) 2,2-Dimethyl-11-äthyl-amino-undeca-4,8-dienal-oxim als hochviskose Flüssigkeit; Ausbeute 87,9% d. Th.; Sdp. 162—164°C/7 Pa.

Analyse für $C_{15}H_{28}N_2O$ (Molgewicht 252,40):

| | | | | |
|---|---|---|---|---|
| berechnet | C 71,38 | H 11,18 | N 11,10 | O 6,34% |
| gefunden | C 71,07 | H 11,33 | N 10,97 | O 6,59% |

MS-Spektrum: Molekül-Peak 252, Bruchstückmassen 223, 208, 166, 140, 58.
$^1$H-NMR-Spektrum $\tau$ (ppm): 2,72 (s), 4,58 (m), 5–7 (m), 7,31 (quin), 7,87 (m), 8,4–8,8 (m), 8,90 (s) und 9,05 (t) im Verhältnis 1 : 4 : 1 : 1 : 8 : 2 : 11.

## Beispiel 4

a) Es wird wie in Beispiel 2a) beschrieben vorgegangen, jedoch unter Verwendung von 438 g (2 Mol) 3,3-Dimethyl-12-äthyl-1-aza-1,5,9-cyclododecatrien. Nach der Destillation erhält man 412 g (1,85 Mol) 3,3-Dimethyl-12-äthyl-1-azacyclododecen; Sdp. 61–63° C/4 Pa; $n_D^{20}$ = 1,4721.
MS-Spektrum: Molekül-Peak 223, Bruchstückmassen 194, 138, 124, 112.
$^1$H-NMR-Spektrum $\tau$ (ppm): 2,53 (s), 7,22 (m), 8,2–8,8 (m), 8,87 (s) und 8,91 (s), 9,19 (t) im Verhältnis 1 : 1 : 18 : 6 : 3.

b) Es wird wie in Beispiel 1b) beschrieben vorgegangen, jedoch unter Verwendung von 223 g (1 Mol) 3,3-Dimethyl-12-äthyl-1-aza-cyclododecen, 82 g (0,5 Mol) Hydroxylaminsulfat, 150 g Schwefelsäure und 400 ml Wasser. Nach der Destillation erhält man 245 g (0,955 Mol) 2,2-Dimethyl-11-äthyl-11-amino-undecanal-oxim; Ausbeute 95,5% d. Th.; Sdp. 155–160° C/7 Pa.
Analyse für $C_{15}H_{32}N_2O$ (Molgewicht 256,43):

| | | | | |
|---|---|---|---|---|
| berechnet | C 70,26 | H 12,58 | N 10,93 | O 6,24% |
| gefunden | C 70,60 | H 12,28 | N 11,00 | O 6,53% |

MS-Spektrum: Molekül-Peak 256, Bruchstückmassen 239, 227, 184, 170, 140, 58.
$^1$H-NMR-Spektrum $\tau$ (ppm): 2,74 (s), 5,6–6,5 (m), 7,35 (m), 8,4–8,8 (m), 8,91 (s), 9,07 (t) im Verhältnis 1 : 1 : 1 : 20 : 6 : 3.

## Beispiel 5

a) Analog Beispiel 1a) werden 122,5 g (0,98 Mol) N-Isobutyliden-2-methylpropenylamin [hergestellt durch Umsetzung von Isobutyraldehyd mit Ammoniak gemäß J. Org. Chem., 26, 1822–25 (1961)] mit 1,3-Butadien umgesetzt. Nach der Destillation erhält man 212,5 g (0,912 Mol) 3,3-Dimethyl-12-isopropyl-1-aza-1,5,9-cyclododecatrien; Sdp. 54–55° C/1,33 Pa; $n_D^{20}$ = 1,4832.

b) Es wird wie in Beispiel 1b) beschrieben vorgegangen, jedoch unter Verwendung von 233,4 g (1 Mol) 3,3-Dimethyl-12-isopropyl-1-aza-1,5,9-cyclododecatrien, 100 g 37%iger Salzsäure, 200 ml Wasser und 69,5 g (1,0 Mol) Hydroxylaminhydrochlorid. Nach der Aufarbeitung und anschließender Destillation erhält man 245 g (0,92 Mol) 2,2-Dimethyl-11-isopropyl-11-amino-undeca-4,8-dienal-oxim; Ausbeute 92% d. Th.; Sdp. 158–162° C/4 Pa; $n_D^{25}$ = 1.4930.
Analyse für $C_{16}H_{30}N_2O$ (Molgewicht 266,43):

| | | | | |
|---|---|---|---|---|
| berechnet | C 72,13 | H 11,35 | N 10,52 | O 6,01% |
| gefunden | C 72,86 | H 11,39 | N 10,21 | O 5,65% |

MS-Spektrum: Molekül-Peak 266, Bruchstückmassen 261, 223, 164, 140, 72.
$^1$H-NMR-Spektrum $\tau$ (ppm): 2,73 (s), 4,6 (m), 5–7 (m), 7,35–7,55 (m), 7,89 (m), 8,2–8,6 (m), 8,90 (s) und 9,05 (d) im Verhältnis 1 : 4 : 1 : 1 : 8 : 1 : 14.

## Beispiel 6

Es wird wie in Beispiel 5b) beschrieben vorgegangen, jedoch unter Verwendung von 300 ml Wasser anstelle von 110 g 37%iger Salzsäure und 200 ml Wasser. Nach einer Reaktionszeit von 45 Minuten bei 100° C und Aufarbeitung wie in 5b) beschrieben erhält man das 2,2-Dimethyl-11-isopropyl-11-amino-undeca-4,8-dienal-oxim in einer Ausbeute von 93% d. Th.

## Beispiel 7

a) Es wird wie in Beispiel 2a) beschrieben vorgegangen, jedoch unter Verwendung von 466,8 g (2 Mol) 3,3-Dimethyl-12-isopropyl-1-aza-1,5,9-cyclododecatrien. Nach der Destillation erhält man 425 g (1,79 Mol) 3,3-Dimethyl-12-isopropyl-1-aza-cyclododecen; Sdp. 92–94° C/4 Pa; $n_D^{20}$ = 1,4706.
MS-Spektrum: Molekül-Peak 237, Bruchstückmassen 222, 194, 138, 126.
$^1$H-NMR-Spektrum $\tau$ (ppm): 2,59 (s), 7,51 (m), 8,1–8,8 (m), 8,87 (s) und 8,90 (s), 9,12 (dd) im Verhältnis 1 : 1 : 17 : 6 : 6.

b) Es wird wie in Beispiel 1b) beschrieben vorgegangen, jedoch unter Verwendung von 118,7 g (0,5 Mol) 3,3-Dimethyl-12-isopropyl-1-aza-cyclododecen, 35 g (0,5 Mol) Hydroxylaminhydrochlorid, 55 g 37%iger Salzsäure, 200 ml Wasser und 50 g (1,25 Mol) festem Natriumhydroxid. Nach der Destillation erhält man 127 g (0,47 Mol) 2,2-Dimethyl-11-isopropyl-11-amino-undecanal-oxim; Ausbeute 94% d. Th.; Sdp. 145° C/4 Pa; $n_D^{20}$ = 1,4761.
Analyse für $C_{16}H_{34}N_2O$ (Molgewicht 270,46):

| | | | | |
|---|---|---|---|---|
| berechnet | C 71,06 | H 12,67 | N 10,36 | O 5,91% |
| gefunden | C 71,13 | H 12,68 | N 10,30 | O 5,91% |

MS-Spektrum: Molekül-Peak 270, Bruchstückmassen 253, 236, 227, 209, 182, 109.
$^1$H-NMR-Spektrum $\tau$ (ppm): 2,62 (s), 5,6 – 6,5 (m), 7,38 (m), 8,1 – 8,75 (m), 8,84 (s), 9,03 (dd) im Verhältnis 1 : 1 : 1 : 19 : 6 : 6.

Beispiel 8

a) Es wird wie unter 1a) beschrieben vorgegangen, jedoch unter Verwendung von 110 g (1,13 Mol) N-Isopropyliden-propenylamin [hergestellt durch Umsetzung von Aceton mit Allylamin und anschließende Isomerisierung; vgl. Zhurnal Organicheskoi Khimii, 6, Nr. 11, 2197 – 9 (1970)] und 108 g (2 Mol) 1,3-Butadien. Nach der Destillation erhält man 187,0 g (0,91 Mol) 3,12,12-Trimethyl-1-aza-1,5,9-cyclododecatrien, Sdp: 55° C/4 Pa; $n_D^{20}$ = 1,4985.

b) Es wird wie in Beispiel 1b) beschrieben vorgegangen, jedoch unter Verwendung von 205 g (1 Mol) 3,12,12-Trimethyl-1-aza-1,5,9-cyclododecatrien, 82 g (0,5 Mol) Hydroxylaminsulfat, 110 g Schwefelsäure und 300 ml Wasser. Nach der Aufarbeitung und anschließender Destillation erhält man 205 g (0,86 Mol) 2,11,11-Trimethyl-11-amino-undeca-4,8-dienaloxim; Ausbeute 86% d. Th.; Sdp. 155° C/20 Pa; $n_D^{20}$ = 1,4950.
Analyse für $C_{14}H_{26}N_2O$ (Molgewicht 238,38):

| | | | | |
|---|---|---|---|---|
| berechnet | C 70,54 | H 11,00 | N 11,75 | O 6,71% |
| gefunden | C 70,69 | H 11,12 | N 11,72 | O 6,74% |

MS-Spektrum: Molekül-Peak 238, Bruchstückmassen 223, 204, 190, 166, 126, 58.
$^1$N-NMR-Spektrum $\tau$ (ppm): 2,75 (d), 4,58 (m), 5 – 7 (m), 7,58 (quin), 7,9 (m), 8,87 (s) und 8,95 (d) im Verhältnis 1 : 4 : 1 : 1 : 10 : 9.

Beispiel 9

a) Es wird wie in Beispiel 1a) beschrieben vorgegangen, jedoch unter Verwendung von 678 g (3,31 Mol) N-Cyclohexyl-methyliden-(cyclohexyliden-methylamin) [hergestellt durch Umsetzung von Cyclohexanaldehyd mit Ammoniak; Sdp. 83° C/4 Pa; $n_D^{20}$ = 1,5260]. Nach der Destillation erhält man 851 g (2,72 Mol) 3-Pentamethylen-12-cyclohexyl-1-aza-1,5,9-cyclododecatrien; Sdp. 140° C/3 Pa; $n_D^{20}$ = 1,5191.

b) Es wird wie in Beispiel 2a) beschrieben vorgegangen, jedoch unter Verwendung von 750 g (2,4 Mol) 3-Pentamethylen-12-cyclohexyl-1-aza-1,5,9-cyclododecatrien. Nach der Destillation erhält man 733,9 g (2,32 Mol) 3-Pentamethylen-12-cyclohexyl-1-aza-cyclododecen; Ausbeute 96,6% d. Th.; Sdp. 140 – 142° C/3 Pa; $n_D^{20}$ = 1,4982.
MS-Spektrum: Molekül-Peak 317, Bruchstückmassen 234, 206, 138.
$^1$H-NMR-Spektrum $\tau$ (ppm): 2,72 (s), 7,5 (m), 8,0 – 9,0 (m) im Verhältnis 1 : 1 : 37.

c) Es wird wie in Beispiel 1b) beschrieben vorgegangen, jedoch unter Verwendung von 317 g (1 Mol) 3-Pentamethylen-12-cyclohexyl-1-aza-cyclododecen, 70 g (1 Mol) Hydroxylaminhydrochlorid, 100 g 37%iger Salzsäure, 300 ml Wasser und 100 g festem Natriumhydroxid. Die sich abscheidende organische Phase enthält 340 g (0,97 Mol) 2-Pentamethylen-11-cyclohexyl-11-amino-undecanal-oxim; Ausbeute 97% d. Th.

Beispiel 10

a) Es wird wie in Beispiel 1a) beschrieben vorgegangen, jedoch unter Verwendung von 1010 g (6,35 Mol) N-Benzyliden-(2-methylpropenylamin) [hergestellt durch Umsetzung von Benzaldehyd mit Methallylamin und anschließende Isomerisierung in Gegenwart von Kalium-tert.-butylat; Sd. 65 – 66° C/7 Pa; $n_D^{20}$ = 1,5836]. Nach einer Reaktionszeit von 2 Stunden bei 85° C und anschließender Destillation erhält man 1398 g (5,24 Mol) 3,3-Dimethyl-12-phenyl-1-aza-1,5,9-cyclododecatrien; Sdp. 128 – 130° C/4 Pa; Sdp. 66 – 68° C.

b) Es wird wie in Beispiel 1b) beschrieben vorgegangen, jedoch unter Verwendung von 289,3 g (1,09 Mol) 3,3-Dimethyl-12-phenyl-1-aza-1,5,9-cyclododecatrien, 88,7 g (0,54 Mol) Hydroxylaminsulfat, 100 g 37%iger Salzsäure, 400 ml Wasser und 100 g festem Natriumhydroxid. Die sich abscheidende organische Phase enthält 320 g (1,065 Mol) 2,2-Dimethyl-11-phenyl-11-amino-undeca-4,8-dienal-oxim; Ausbeute 97,9% d. Th.

## Beispiel 11

a) Es wird wie in Beispiel 1a) beschrieben vorgegangen, jedoch unter Verwendung von 273 g (2,02 Mol) N-2-Furylidenpropenylamin [hergestellt durch Umsetzung von 2-Furylaldehyd mit Allylamin und anschließende Isomerisierung in Gegenwart von Kalium-tert-butylat; Sdp. 60–62°C/67 Pa; $n_D^{20}$ = 1,6004]. Nach der Destillation erhält man 210 g (0,865 Mol) 3-Methyl-12-(2-furyl)-1-aza-1,5,9-cyclododecatrien; Sdp. 106–108°C/4 Pa; $n_D^{20}$ = 1,5260.

b) Es wird wie in Beispiel 1b) beschrieben vorgegangen, jedoch unter Verwendung von 190 g (0,78 Mol) 3-Methyl-12-(2-furyl)-1-aza-1,5,9-cyclododecatrien, 54 g (0,78 Mol) Hydroxylaminhydrochlorid, 84 g Schwefelsäure, 300 ml Wasser und 65 g festem Natriumhydroxid. Die sich abscheidende organische Phase enthält 201 g (0,728 Mol) 2-Methyl-11-(2-furyl)-11-amino-undeca-4,8-dienal-oxim; Ausbeute 93% d. Th.; $n_D^{20}$ = 1,5305.

Analyse für $C_{16}H_{24}N_2O_2$ (Molgewicht 276,38):

| | | | | |
|---|---|---|---|---|
| berechnet | C 69,53 | H 8,75 | N 10,14 | O 11,58% |
| gefunden | C 71,00 | H 8,73 | N 9,11 | O 11,18% |

MS-Spektrum: Molekül-Peak 276, Bruchstückmassen 259, 242, 228, 204, 148, 133, 96.
$^1$H-NMR-Spektrum $\tau$ (ppm): 2,7–2,9 (m), 3,7–3,9 (m), 4,6 (m), 6,03 (t), 7,4–8,0 (m), 9,0 (dd) im Verhältnis 2 : 2 : 4 : 1 : 12 : 3.

## Beispiel 12

a) Es wird wie in Beispiel 1a) beschrieben vorgegangen, jedoch unter Verwendung von 629 g (3,55 Mol) N-Cyclopentylmethyliden-(cyclopentylidenmethylamin) [hergestellt durch Umsetzung von Cyclopentanaldehyd mit Ammoniak; Sdp. 125°C/1,86 × 10³ Pa; $n_D^{20}$ = 1,5245]. Nach der Destillation erhält man 789 g (2,8 Mol) 3-Tetramethylen-12-cyclopentyl-1-aza-1,5,9-cyclododecatrien; Sdp. 120°C/1 Pa.

b) Es wird wie in Beispiel 2a) beschrieben vorgegangen, jedoch unter Verwendung von 500 g (1,75 Mol) 3-Tetramethylen-12-cyclopentyl-1-aza-1,5,9-cyclododecatrien. Nach der Destillation erhält man 470 g (1,63 Mol) 3-Tetramethylen-12-cyclopentyl-1-aza-cyclododecen; Ausbeute 93% d. Th.; Sdp. 130°C/7 Pa. MS-Spektrum: Molekül-Peak 289, Bruchstückmassen 220, 178, 142, 98.

c) Es wird wie in Beispiel 2b) beschrieben vorgegangen, jedoch unter Verwendung von 470 g (1,63 Mol) 3-Tetramethylen-12-cyclopentyl-1-aza-cyclododecen, 133 g (0,81 Mol) Hydroxylaminsulfat und 162 g 37%iger Salzsäure sowie 400 ml Wasser. Nach der Neutralisation mit festem Natriumhydroxid erhält man 525 g (1,63 Mol) 2-Tetramethylen-11-cyclopentyl-11-amino-undecanal-oxim; Ausbeute 100% d. Th.

## Beispiel 13

a) Analog der in den vorangehenden Beispielen beschriebenen Arbeitsweise werden 710 g (3,93 Mol) N-2-Methyl-pentyliden-(2-methyl-penten-1-yl-amin) [hergestellt durch Umsetzung von 2-Methylvaleraldehyd mit Ammoniak gemäß US-PS 2 319 848] und 432 g (8,0 Mol) 1,3-Butadien umgesetzt. Nach der Aufarbeitung des Reaktionsgemisches erhält man 995 g (3,45 Mol) 3-Methyl-3-n-propyl-12-(2-pentyl)-1-aza-1,5,9-cyclododecatrien als Isomerengemisch (2 Hauptisomere); Sdp. 103–105°C/40 Pa; $n_D^{20}$ = 1,4886.

b) Es wird wie in Beispiel 2a) beschrieben vorgegangen, jedoch unter Verwendung von 289,5 g (1 Mol) 3-Methyl-3-n-propyl-12-(2-pentyl)-1-aza-1,5,9-cyclododecatrien. Nach der Destillation erhält man 263 g (0,896 Mol) 3-Methyl-3-n-propyl-12-(2-pentyl)-1-aza-cyclododecen; Sdp. 125°C/53 Pa.

c) Es wird wie in Beispiel 2b) beschrieben vorgegangen, jedoch unter Verwendung von 293,55 g (1 Mol) 3-Methyl-3-n-propyl-12-(2-pentyl)-1-aza-cyclododecen, 82,1 g (0,5 Mol) Hydroxylaminsulfat, 100 g 37%iger Salzsäure, 400 ml Wasser und 85 g festem Natriumhydroxid. Die sich abscheidende organische Phase enthält 325 g (0,996 Mol) 2-Methyl-2-n-propyl-11-(2-pentyl)-11-amino-undecanal-oxim; Ausbeute 99,6% d. Th.

Beispiel 14

a) Es wird wie in den vorangehenden Beispielen beschrieben vorgegangen, jedoch unter Verwendung von 72,4 g (0,4 Mol) 1-(3-Pentyl)-4,4-diäthyl-2-aza-1,3-butadien [hergestellt durch Umsetzung von 2-Äthylbutyraldehyd mit Ammoniak gemäß US-PS 2 319 848] und 48,4 g (0,895 Mol) 1,3-Butadien. Nach der Aufarbeitung erhält man 56,8 g (0,197 Mol) 3,3-Diäthyl-12-(3-pentyl)-1-aza-1,5,9-cyclododecatrien; Sdp. 90 – 92° C/0,13 Pa; $n_D^{20}$ = 1,4840.

b) Es wird wie in Beispiel 2a) beschrieben vorgegangen, jedoch unter Verwendung von 289 g (1 Mol) 3,3-Diäthyl-12-(3-pentyl)-1-aza-1,5,9-cyclododecatrien. Nach der Destillation erhält man 265 g (0,905 Mol) 3,3-Diäthyl-12-(3-pentyl)-1-aza-cyclododecen; Ausbeute 90,5% d. Th.; Sdp. 95° C/4 Pa.

c) Es wird wie in Beispiel 2b) beschrieben vorgegangen, jedoch unter Verwendung von 320 g (1,09 Mol) 3,3-Diäthyl-12-(3-pentyl)-1-aza-cyclodedecen, 89,5 g (0,546 Mol) Hydroxylaminsulfat, 110 g 37%iger Salzsäure und 400 ml Wasser. Nach der Neutralisation mit festem Natriumhydroxid erhält man 350 g (1,07 Mol) 2,2-Diäthyl-11-(3-pentyl)-11-aminoundecanal-oxim; Ausbeute 98% d. Th.; $n_D^{20}$ = 1,4637.

Beispiel 15

a) Es wird wie in den vorangehenden Beispielen beschrieben vorgegangen, jedoch unter Verwendung von 760 g (3,21 Mol) N-2-Äthylhexyliden-(2-äthyl-hexen-1-yl-amin) [hergestellt durch Umsetzung von 2-Äthyl-capronaldehyd mit Ammoniak gemäß US-PS 2 319 848] und 378 g (7 Mol) 1,3-Butadien. Nach der Aufarbeitung des Reaktionsgemisches erhält man 930 g (2,69 Mol) 3-Äthyl-3-n-butyl-12-(3-heptyl)-1-aza-1,5,9-cyclododecatrien als ein 7 : 3-Isomerengemisch; Sdp. 106 – 109° C/13 Pa; $n_D^{20}$ = 1,4895.

b) Es wird wie in Beispiel 2a) beschrieben vorgegangen, jedoch unter Verwendung von 396 g (1,15 Mol) 3-n-Butyl-3-äthyl-12-(3-heptyl)-1-aza-1,5,9-cyclododecatrien. Nach der Destillation erhält man 384 g (1,1 Mol) 3-n-Butyl-3-äthyl-12-(3-heptyl)-1-aza-cyclododecen; Ausbeute 95,6% d. Th.; Sdp. 130° C/4 Pa.

c) Es wird wie in Beispiel 2b) beschrieben vorgegangen, jedoch unter Verwendung von 300 g (0,859 Mol) 3-n-Butyl-3-äthyl-12-(3-heptyl)-1-aza-cyclododecen, 70,3 g (0,43 Mol) Hydroxylaminsulfat und 85 g 37%iger Salzsäure sowie 400 ml Wasser. Nach der Neutralisation mit festem Natriumhydroxid erhält man 329 g (0,86 Mol) 2-n-Butyl-2-äthyl-11-(3-heptyl)-11-amino-undecanal-oxim; Ausbeute 100% d. Th.

Beispiel 16

a) Es wird wie in den vorangehenden Beispielen beschrieben vorgegangen, jedoch unter Verwendung von 467 g (2,8 Mol) N-Propyliden-(2-äthyl-hexen-1-yl-amin), 324 g (6 Mol) 1,3-Butadien, 15,7 g (61 mMol) Nickelacetylacetonat, 7,45 g (60 mMol) Trimethylphosphit, 23,4 g (180 mMol) Äthoxy-diäthylaluminium und 300 ml Toluol. Nach einer Reaktionszeit von 4 Stunden bei 40° C erhält man nach der Aufarbeitung 624 g (2,27 Mol) 3,12-Diäthyl-3-n-butyl-1-aza-1,5,9-cyclododecatrien als Isomerengemisch; Ausbeute 81% d. Th.; Sdp. 98 – 100° C/40 Pa; $n_D^{20}$ = 1,4905.

Das im obigen Beispiel verwendete N-Propyliden-(2-äthyl-hexen-1-yl-amin) wurde auf analoge Weise wie das N-Propyliden-(2-methylpropenylamin) gemäß Beispiel 3a) hergestellt, jedoch unter Verwendung von 10 g Kalium-tert-butylat, 800 g (4,79 Mol) (2-Äthyl-hexyliden)-allylamin und 600 ml Tetrahydrofuran. Nach einer Reaktionszeit von 2 Stunden bei 35° C erhält man 682 g (4,08 Mol) N-Propyliden-(2-äthylhexen-1-yl-amin); Isomerengemisch im Gewichtsverhältnis von 55 : 45; Sdp. 53 – 56° C/133 Pa; $n_D^{20}$ = 1,4698.

b) Es wird wie in Beispiel 2a) beschrieben vorgegangen, jedoch unter Verwendung von 275,5 g (1 Mol) 3-n-Butyl-3,12-diäthyl-1-aza-1,5,9-cyclododecatrien. Nach der Destillation erhält man 245,5 g (0,878 Mol) 3-n-Butyl-3,12-diäthyl-1-aza-cyclododecen; Ausbeute 87,8% d. Th.; Sdp. 110° C/7 Pa.

c) Es wird wie in Beispiel 2b) beschrieben vorgegangen, jedoch unter Verwendung von 245,5 g (0,878 Mol) 3-n-Butyl-3,12-diäthyl-1-aza-cyclododecen, 74 g (0,45 Mol) Hydroxylaminsulfat, 100 g 37%iger Salzsäure und 200 ml Wasser. Nach der Neutralisation mit festem Natriumhydroxid erhält man 255 g (0,815 Mol) 2-n-Butyl-2,11-diäthyl-11-amino-undecanal-oxim; Ausbeute 92,9% d. Th.

Beispiel 17

a) Es wird wie in Beispiel 1a) beschrieben vorgegangen, jedoch unter Verwendung von 302,5 g (2 Mol) N-Cyclohexyliden-(2-methylpropenylamin) und 250 g (4,62 Mol) 1,3-Butadien. Durch Destillation erhält man 382 g (1,48 Mol) 3,3-Dimethyl-12-pentamethylen-1-aza-1,5,9-cyclododecatrien; Sdp. 96° C/4 Pa; $n_D^{20}$ = 1,5116. Das N-Cyclohexyliden-(2-methylpropenylamin) wurde aus Cyclohexanon und

Methallylamin und anschließende Isomerisierung des Reaktionsproduktes mit Kalium-tert-butylat hergestellt; Sdp. 96° C/1700 Pa; $n_D^{20}$ = 1,5160.

b) Es wird wie in Beispiel 1b) beschrieben vorgegangen, jedoch unter Verwendung von 259,5 g (1 Mol) 3,3-Dimethyl-12-pentamethylen-1-aza-1,5,9-cyclododecatrien, 69,5 g (1 Mol) Hydroxylaminhydrochlorid, 20 g 37%iger Salzsäure und 250 ml Wasser. Nach der Aufarbeitung erhält man 219,3 g 2,2-Dimethyl-11-pentamethylen-11-amino-undeca-4,8-dienal-oxim; Ausbeute 74,5% d. Th.; $n_D^{20}$ = 1,5117.

## Beispiel 18

a) Es wird wie in Beispiel 1a) beschrieben vorgegangen, jedoch unter Verwendung von 94,2 g (0,564 Mol) N-Heptyliden-(2-methylpropenylamin) [1-n-Hexyl-4,4-dimethyl-2-aza-1,3-butadien] und 80 g (1,48 Mol) 1,3-Butadien. Durch Destillation erhält man 113 g (0,41 Mol) 3,3-Dimethyl-12-n-hexyl-1-aza-1,5,9-cyclododecatrien; Sdp. 100° C/4 Pa; $n_D^{20}$ = 1,4841.

Das N-Heptyliden-(2-methylpropenylamin) wurde aus Heptanal und Methallylamin und durch anschließende Isomerisierung des Reaktionsproduktes mit Kalium-tert-butylat hergestellt; Sdp. 54° C/5 Pa; $n_D^{20}$ = 1,4662.

b) Es wird wie in Beispiel 1b) beschrieben vorgegangen, jedoch unter Verwendung von 113 g (0,41 Mol) 3,3-Dimethyl-12-hexyl-1-aza-1,5,9-cyclododecatrien, 33 g (0,202 Mol) Hydroxylaminsulfat, 50 g konz. Salzsäure und 250 ml Wasser. Nach der Aufarbeitung erhält man 125 g (0,405 Mol) 2,2-Dimethyl-11-n-hexyl-11-amino-undeca-4,8-dienal-oxim; Ausbeute 99% d. Th.

## Beispiel 19

a) Es wird wie in Beispiel 1a) beschrieben vorgegangen, jedoch unter Verwendung von 222,4 g (2,0 Mol) N-Isopropyliden-(2-methylpropenylamin). Durch Destillation erhält man 300 g (1,37 Mol) 3,3,12,12-Tetramethyl-1-aza-1,5,9-cyclododecatrien; Sdp. 58° C/4 Pa; $n_D^{20}$ = 1,4858. Das N-Isopropyliden-(2-methylpropenylamin) wurde aus Aceton und Methallylamin und durch anschließende Isomerisierung des Reaktionsproduktes hergestellt; Sdp. 89 – 90° C; $n_D^{20}$ = 1,4762.

b) Es wird wie in Beispiel 1b) beschrieben vorgegangen, jedoch unter Verwendung von 110 g (0,5 Mol) 3,3,12,12-Tetramethyl-1-aza-1,5,9-cyclododecatrien, 41 g (0,25 Mol) Hydroxylaminsulfat, 50 ml konz. Salzsäure und 250 ml Wasser. Nach der Destillation erhält man 85 g (0,337 Mol) 2,2,11,11-Tetramethyl-11-amino-undeca-4,8-dienal-oxim; Ausbeute 67,4% d. Th.; Sdp. 130° C/7 Pa.

## Beispiel 20

a) Es wird wie in Beispiel 1a) beschrieben vorgegangen, jedoch unter Verwendung von 93,6 g (0,5 Mol) N-Benzyliden-(2-äthyl-1-butenylamin) [1-Phenyl-4,4-diäthyl-2-aza-1,3-butadien, hergestellt aus Benzylamin und 2-Äthyl-butenal und durch anschließende Isomerisierung des Reaktionsproduktes in Gegenwart von Kalium-tert-butylat; Sdp. 70° C/7 Pa; $n_D^{20}$ = 1,5598; vgl. J. Org. Chem., 43, Nr. 4, 782 – 84 (1978)]. Nach der Destillation erhält man 116 g (0,393 Mol) 3,3-Diäthyl-12-phenyl-1-aza-1,5,9-cyclododecatrien; Sdp. 105° C/4 Pa; $n_D^{20}$ = 1,5369.

b) Es wird wie in Beispiel 1b) beschrieben vorgegangen, jedoch unter Verwendung von 95,3 g (0,323 Mol) 3,3-Diäthyl-12-phenyl-1-aza-1,5,9-cyclododecatrien, 20 g 37%iger Salzsäure, 22,4 g (0,322 Mol) Hydroxylaminhydrochlorid und 250 ml Wasser. Nach der Aufarbeitung erhält man 103,1 g (0,314 Mol) 2,2-Diäthyl-11-phenyl-amino-undeca-4,8-dienal-oxim; Ausbeute 97,5% d. Th.

## Beispiel 21

a) Es wird wie in Beispiel 1a) beschrieben vorgegangen, jedoch unter Verwendung von 215,3 g (1 Mol) N-Benzyliden-(2-äthyl-1-hexenylamin) [1-Phenyl-4-äthyl-4-n-butyl-2-aza-1,3-butadien; hergestellt durch Umsetzung von Benzylamin mit 2-Äthyl-hexenal und anschließende Isomerisierung des Reaktionsproduktes mit Kalium-tert-butylat; Sdp. 90° C/7 Pa; $n_D^{20}$ = 1,5630]. Nach der Destillation erhält man 288 g (0,891 Mol) 3-n-Butyl-3-äthyl-12-phenyl-1-aza-1,5,9-cyclododecatrien; Sdp. 130° C/2 Pa; $n_D^{20}$ = 1,5296.

b) Es wird wie in Beispiel 1b) beschrieben vorgegangen, jedoch unter Verwendung von 283,4 g (0,875 Mol) 3-n-Butyl-3-äthyl-12-phenyl-1-aza-1,5,9-cyclododecatrien, 60,8 g (0,87 Mol) Hydroxylaminhydrochlorid, 50 ml konz. Salzsäure und 250 ml Wasser. Nach der Aufarbeitung erhält man 310 g (0,87 Mol) 2-n-Butyl-2-äthyl-11-phenyl-11-amino-undeca-4,8-dienal-oxim; Ausbeute 99,5% d. Th.

Beispiel 22

a) Es wird wie in Beispiel 1a) beschrieben vorgegangen, jedoch unter Verwendung von 184 g (1,34 Mol) N-(2-Äthyl)-buten-2-yliden-propenylamin [hergestellt durch Umsetzung von 2-Äthyl-butenal und Allylamin und anschließende Isomerisierung des Reaktionsproduktes analog Zhurnal Organicheskoi Khimii, 6, Nr. 11, 2197—9 (1970); Sdp. 70°C/1700 Pa; $n_D^{20}$ =1,5227]. Nach der Destillation erhält man 295 g (1,21 Mol) 3-Methyl-12-(3-penten-2-yl)-1-aza-1,5,9-cyclododecatrien; Sdp. 100°C/4 Pa; $n_D^{20}$ =1,5056.

b) Es wird wie in Beispiel 1b) beschrieben vorgegangen, jedoch unter Verwendung von 122,7 g (0,5 Mol) 3-Methyl-12-(3-penten-2-yl)-1-aza-1,5,9-cyclododecatrien, 41,1 g (0,25 Mol) Hydroxylaminsulfat, 50 ml konz. Salzsäure und 250 ml Wasser. Nach der Aufarbeitung erhält man 135,9 g (0,488 Mol) 2-Methyl-11-(3-penten-2-yl)-11-amino-undeca-4,8-dienal-oxim; Ausbeute 97,6% d. Th.; $n_D^{20}$ =1,5091.

Beispiel 23

a) Es wird wie in Beispiel 1a) beschrieben vorgegangen, jedoch unter Verwendung von 105 g (0,765 Mol) N-(2-Äthyl)-buten-2-yliden-propenylamin und 120 g (1,76 mMol) Isopren. Nach einer Reaktionszeit von 5 Stunden bei 90°C erhält man bei der anschließenden Destillation 85 g (0,312 Mol) 3,5- (bzw. 6), 9- (bzw. 10) -Trimethyl-12,(3-penten-2-yl)-1-aza-1,5,9-cyclododecatrien; Sdp. 108 bis 110°C/5 Pa; $n_D^{20}$ =1,5078.

b) Es wird wie in Beispiel 1b) beschrieben vorgegangen, jedoch unter Verwendung von 83,75 g (0,305 Mol) des obigen 1-Aza-1,5,9-cyclododecatriens, 26,8 g (0,163 Mol) Hydroxylaminsulfat, 35 ml konz. Salzsäure und 250 ml Wasser. Nach der Aufarbeitung erhält man 90 g (0,294 Mol) 2,4(5),8(9)-Trimethyl-11-(3-penten-2-yl)-11-aminoundeca-4,8-dienal-oxim; Ausbeute 96,5% d. Th.

Beispiel 24

Es wird wie in Beispiel 1b) beschrieben vorgegangen, jedoch unter Verwendung von 23,3 g (0,1 Mol) 3,3-Dimethyl-12-isopropyl-1-aza-1,5,9-cyclododecatrien, 20 g 37%iger Salzsäure, 20 ml Wasser und 10,8 g (0,1 Mol) Phenylhydrazin. Nach der Aufarbeitung wie in Beispiel 1b) beschrieben, erhält man 34,1 g (0,1 Mol) 2,2-Dimethyl-11-isopropyl-11-amino-undeca-4,8-dienal-phenylhydrazon; Ausbeute 100% d. Th.
Analyse für $C_{22}H_{35}N_3$ (Molgewicht 341,54):

| | | | |
|---|---|---|---|
| berechnet | C 77,37 | H 10,33 | N 12,30% |
| gefunden | C 77,03 | H 10,58 | N 12,23% |

MS-Spektrum: Molekül-Peak 341, Bruchstückmassen 298, 270, 234, 161, 92, 72.
$^1$H-NMR-Spektrum $\tau$ (ppm): 2,65—3,3 (m), 4,58 (m), 7,45 (dt), 7,89 (m), 8,03 (s), 8,35 (m), 8,88 (s), 9,06 (dd) im Verhältnis 7 : 4 : 1 : 8 : 1 : 6 : 6.

Beispiel 25

Es wird wie in Beispiel 1b) beschrieben vorgegangen, jedoch unter Verwendung von 46,6 g (0,2 Mol) 3,3-Dimethyl-12-isopropyl-1-aza-1,5,9-cyclododecatrien, 40 g 37%iger Salzsäure, 40 ml Wasser und 5 g (0,1 Mol) Hydrazinhydrat. Nach der Aufarbeitung erhält man 42 g (0,84 Mol) Di-(2,2-dimethyl-11-isopropyl-11-aminoundeca-4,8-dienal)-hydrazon; Ausbeute 84% d. Th.
Analyse für $C_{32}H_{58}N_4$ (Molgewicht 498,84):

| | | | |
|---|---|---|---|
| berechnet | C 77,05 | H 11,72 | N 11,23% |
| gefunden | C 77,83 | H 12,08 | N 10,84% |

MS-Spektrum: Molekül-Peak 498, Bruchstückmassen 483, 455, 438, 426, 372, 318, 276.
$^1$H-NMR-Spektrum $\tau$ (ppm): 2,38 (s), 4,58 (m), 7,5 (m), 7,88 (m), 8,1—8,7 (m), 8,88 (s), 9,06 (dd) im Verhältnis 2 : 8 : 2 : 16 : 6 : 12 : 12.

Beispiel 26

Es wird wie in Beispiel 1b) beschrieben vorgegangen, jedoch unter Verwendung von 23,3 g (0,1 Mol) 3,3-Dimethyl-12-isopropyl-1-aza-1,5,9-cyclododecatrien, 20 g 37%iger Salzsäure, 20 ml Wasser und

11,15 g (0,1 Mol) Semicarbazidhydrochlorid. Nach der Aufarbeitung erhält man 26,5 g (0,086 Mol) 2,2-Dimethyl-11-isopropyl-11-amino-undeca-4,8-dienal-semicarbazon; Ausbeute 86% d. Th.
Analyse für $C_{17}H_{32}N_4O$ (Molgewicht 308,47):

| | | | | |
|---|---|---|---|---|
| berechnet | C 66,19 | H 10,46 | N 18,16 | O 5,19% |
| gefunden | C 68,5 | H 10,5 | N 17,4 | O 5,0% |

MS-Spektrum Molekül-Peak 308, Bruchstückmassen 265, 248, 205, 182, 129, 72.
$^1$H-NMR-Spektrum $\tau$ (ppm): 0,9 (m), 2,98 (s), 4,32 (s), 4,58 (m), 7,48 (dt), 7,9 (m), 8,1 – 8,8 (m), 8,91 (s), 9,06 (dd) im Verhältnis 1 : 1 : 2 : 4 : 1 : 8 : 3 : 6 : 6.

Beispiel 27

a) Es wird wie in Beispiel 2b) beschrieben vorgegangen, jedoch unter Verwendung von 68,5 g (0,5 Mol) 3,3-Dimethyl-12-isopropyl-1-aza-cyclododecen, 50 g 37%ige Salzsäure, 54 g (0,5 Mol) Benzylamin und 200 ml Wasser. Nach der Neutralisation mit 22 g (0,55 Mol) festem Natriumhydroxid erhält man 97 g (0,282 Mol) 2,2-Dimethyl-11-isopropyl-11-amino-undecanal-benzylamin; Ausbeute 56,4% d. Th.
Analyse für $C_{23}H_{40}N_2$ (Molgewicht 344,59):

| | | | |
|---|---|---|---|
| berechnet | C 80,17 | H 11,70 | N 8,13% |
| gefunden | C 80,0 | H 12,1 | N 7,7% |

MS-Spektrum: Molekül-Peak 344, Bruchstückmassen 301, 253, 161, 91.
$^1$H-NMR-Spektrum $\tau$ (ppm): 2,7 (m), 6,18 (s), 7,3 – 9,2 (m) im Verhältnis 6 : 2 : 32.

Überführung in Diamine der Formel VIII

490 g (1,84 Mol) des gemäß Beispiel 5 erhaltenen 2,2-Dimethyl-11-isopropyl-11-amino-undeca-4,8-dienal-oxims werden in 2,4 Liter Methanol gelöst und zusammen mit ca. 200 g flüssigem Ammoniak unter Zugabe von 150 g Raney-Nickel in einen 6,3-Liter-Stahlautoklav eingefüllt. Es wird anschließend Wasserstoff bis zu einem Druck von 100 bar aufgepreßt und unter Rühren auf 100°C aufgeheizt. Man hydriert ca. 5 Stunden unter diesen Bedingungen, kühlt ab und läßt dann den Ammoniak und überschüssigen Wasserstoff abblasen. Bei der anschließenden Destillation im Hochvakuum erhält man 436 g (1,705 Mol) 1-Isopropyl-10,10-dimethyl-1,11-diaminoundecan als farblose, wasserklare Flüssigkeit; Sdp. 87°C/4 Pa; $n_D^{20}$ = 1,4619.

Auf analoge Weise kann das gemäß Beispiel 7 hergestellte 2,2-Dimethyl-11-isopropyl-11-amino-undecanal-oxim zum 1-Isopropyl-10,10-dimethyl-1,11-diaminoundecan hydriert werden.

Verwendet man im obigen Beispiel anstelle des 2,2-Dimethyl-11-isopropyl-11-amino-undeca-4,8-dienal-oxims 34,1 g (0,1 Mol) 2,2-Dimethyl-11-isopropyl-11-amino-undeca-4,8-dienal-phenylhydrazin (hergestellt gemäß Beispiel 24), 42 g (0,084 Mol) Di-(2,2-dimethyl-11-isopropyl-11-amino-undeca-4,8-dienal)hydrazon (hergestellt gemäß Beispiel 25), 26,5 g (0,086 Mol) 2,2-Dimethyl-11-isopropyl-11-amino-undeca-4,8-dienal-semicarbazon (hergestellt gemäß Beispiel 26) oder 2,2-Dimethyl-11-isopropyl-11-amino-undecanal-benzylamin (hergestellt gemäß Beispiel 27), so erhält man das 1-Isopropyl-10,10-dimethyl-1,11-diamino-undecan in einer Ausbeute von 76% d. Th., 60,6% d. Th., 62,6% d. Th. bzw. 72% d. Th.

Auf die oben beschriebene Weise wurden die folgenden Diamine hergestellt:

1-Methyl-10-phenyl-1,11-diaminoundecan; Sdp. 138 – 140°C/1 Pa;
$n_D^{20}$ = 1,5095 (Oxim gemäß Beispiel 1 bzw. 2);
1-Äthyl-10,10-dimethyl-1,11-diaminoundecan; Sdp. 93°C/7 Pa;
$n_D^{20}$ = 1,4622 (Oxim gemäß Beispiel 3 bzw. 4);
1,1,10-Trimethyl-1,11-diaminoundecan; Sdp. 87°C/4 Pa;
$n_D^{20}$ = 1,4585 (Oxim gemäß Beispiel 8);
1-Cyclohexyl-10-pentamethylen-1,11-diaminoundecan; Sdp. 166 – 170°C/3 Pa;
$n_D^{20}$ = 1,4975 (Oxim gemäß Beispiel 9);
1-Phenyl-10,10-dimethyl-1,11-diaminoundecan; Sdp. 150°C/3 Pa;
$n_D^{20}$ = 1,5054 (Oxim gemäß Beispiel 10);
1-(2-Furyl)-10-methyl-1,11-diaminoundecan; Sdp. 135 – 138°C/7 Pa;
$n_D^{20}$ = 1,4869 (Oxim gemäß Beispiel 11);
1-Cyclopentyl-10-tetramethylen-1,11-diaminoundecan; Sdp. 166 – 168°C/5 Pa;
$n_D^{20}$ = 1,4922 (Oxim gemäß Beispiel 12);
1-(2-Pentyl)-10-methyl-10-n-propyl-1,11-diaminoundecan; Sdp. 140 – 142°C/3 Pa;
$n_D^{20}$ = 1,4665 (Oxim gemäß Beispiel 13);

1-(3-Pentyl)-10,10-diäthyl-1,11-diaminoundecan; Sdp. 133–135° C/3 Pa; $n_D^{20}$ = 1,4704 (Oxim gemäß Beispiel 14);
1-(3-Heptyl)-10-n-butyl-10-äthyl-1,11-diaminoundecan; Sdp. 156–160° C/4 Pa; $n_D^{20}$ = 1,4672 (Oxim gemäß Beispiel 15);
1,10-Diäthyl-10-n-butyl-1,11-diaminoundecan; Sdp. 128–130° C/5 Pa; $n_D^{20}$ = 1,4630 (Oxim gemäß Beispiel 16);
1-Pentamethylen-10,10-diäthyl-1,11-diaminoundecan; Sdp. 112° C/4 Pa; $n_D^{20}$ = 1,4833 (Oxim gemäß Beispiel 17);
1-n-Hexyl-10,10-dimethyl-1,11-diaminoundecan; Sdp. 135° C/4 Pa; $n_D^{20}$ = 1,4624;
1,1,10,10-Tetramethyl-1,11-diaminoundecan; Sdp. 92° C/5 Pa; $n_D^{20}$ = 1,4590;
1-Phenyl-10,10-diäthyl-1,11-diaminoundecan; Sdp. 146° C/2 Pa; $n_D^{20}$ = 1,5090;
1-Phenyl-10-n-butyl-10-äthyl-1,11-diaminoundecan; Sdp. 155–158° C/5 Pa; $n_D^{20}$ = 1,5045;
1-(3-Pentyl)-10-methyl-1,11-diaminoundecan; Sdp. 115° C/5 Pa; $n_D^{20}$ = 1,4662;
1-(3-Pentyl)-3(4),7(8),10-trimethyl-1,11-diaminoundecan; Sdp. 117° C/2 Pa; $n_D^{20}$ = 1,4731.

## Anwendungsbeispiele

### Beispiel A

In einem Kolben, der mit Rührer, Tropftrichter und Rückflußkühler versehen ist, werden 54,5 g Terephthalsäure in einem Gemisch von 750 ml Äthanol und 750 ml Wasser aufgeschlämmt und zum Rückfluß erhitzt. Nun werden aus dem Tropftrichter 103,2 g 1-(3-Pentyl)-10,10-diäthyl-1,11-diaminoundecan dazugegeben. Nach 20 Minuten wird langsam auf Raumtemperatur (20–25° C) abgekühlt, und das ausgefallene Salz wird abfiltriert. Nach dem Trocknen im Vakuum erhält man 147 g Salz (93% d. Th.). 10 g dieses Salzes werden unter Stickstoff in ein Bombenrohr eingeschmolzen und eine Stunde in einem Salzbad auf 270° C erhitzt. Das Salz schmilzt dabei zu einer farblosen Schmelze. Nach dem Abkühlen auf Raumtemperatur wird die erstarrte Schmelze aus dem Bombenrohr entnommen und in einer offenen Polykondensationsapparatur unter Ausschluß von Luft und ständigem Durchleiten von Stickstoff 6 Stunden bei 270° C gehalten. Beim Abkühlen erstarrt die viskose Schmelze zu einer glasklaren, farblosen Masse. Die reduzierte Lösungsviskosität des erhaltenen Polyamids, gemessen als 0,5%ige Lösung in m-Kresol bei 25° C, beträgt 0,91 dl/g; Glasumwandlungstemperatur, bestimmt im Differentialkalorimeter (DSC) = 123° C.

Eine bei 270° C mittels einer hydraulischen Presse hergestellte Folie wird bei Raumtemperatur einer relativen Luftfeuchtigkeit von 65% ausgesetzt, bis keine Gewichtszunahme mehr festzustellen ist. Der Sättigungswert beträgt 0,7 Gewichtsprozent. Setzt man die Folie der Einwirkung von kochendem Wasser aus, so ist nach 6 Stunden noch keinerlei Beeinträchtigung der Transparenz festzustellen.

### Beispiel B

In einem Rundkolben, der mit Rührer, Rückflußkühler und Tropftrichter versehen ist, werden 0,1 Mol Terephthalsäure in 300 ml 70%igem Äthanol zum Sieden erhitzt. In die kochende Suspension läßt man unter Rühren 0,1 Mol 1-Cyclohexyl-10-pentamethylen-1,11-diaminoundecan im Verlaufe von ca. 10 Minuten aus dem Tropftrichter einfließen und spült darin haftende Diaminreste mit etwas Äthanol quantitativ in das Reaktionsgemisch. Die dabei entstandene klare Lösung läßt man unter ständigem Rühren erkalten, filtriert das dabei ausgefallene Salz ab und trocknet es im Vakuum bei 90° C.

In ein Bombenrohr, welches mit einem Schraubdeckel mit eingebautem Überdruckventil ausgestattet ist, werden folgende Komponenten eingewogen:

2,156 g 4,4'-Diamino-3,3'-dimethyldicyclohexylmethan,
1,502 g Isophthalsäure,
8,535 g Salz aus 1-Cyclohexyl-10-pentamethylen-1,11-diaminoundecan und Terephthalsäure.

Nachdem man die Luft im Bombenrohr vollständig durch Stickstoff verdrängt hat, verschließt man das Bombenrohr und taucht es in ein Salzbad, dessen Temperatur 270° C beträgt. Nach 30–60 Minuten ist eine homogene, glasklare Schmelze entstanden. Nach insgesamt 3 Stunden wird die Vorkondensation abgebrochen, indem man das Bombenrohr aus dem Salzbad entfernt und den Überdruck durch Öffnen des Ventils abläßt. Das erstarrte glasklare Vorkondensat wird aus dem

Bombenrohr entfernt und in ein Kondensationsgefäß übergeführt. Unter strengem Ausschluß von Luft und unter dauerndem Durchleiten von Stickstoff wird die Schmelze bei 280°C Salzbadtemperatur 5 Stunden polykondensiert, wobei das Reaktionswasser durch den Stickstoffstrom laufend entfernt wird. Beim Abkühlen erstarrt die Schmelze zu einer glasklaren Masse.

2 – 3 g des hergestellten Copolyamids werden in einer beheizbaren hydraulischen Presse bei 270°C zu einer Folie von ca. 0,4 mm bis 1 mm Dicke verpreßt. Zur Bestimmung der Wasseraufnahme wird die Folie bei Raumtemperatur einer relativen Luftfeuchtigkeit von 65% ausgesetzt, bis keine Gewichtszunahme mehr festzustellen ist. Der Sättigungswert beträgt 1,2 Gewichtsprozent. Die reduzierte Lösungsviskosität des Copolyamids, gemessen als 0,5%ige Lösung in m-Kresol bei 25°C, beträgt 1,09 dl/g; Glasumwandlungstemperatur, bestimmt im Differentialkalorimeter (DSC) = 166°C. Die Beständigkeit der Transparenz gegenüber kochendem Wasser ist sehr gut, das heißt, auch nach mehreren Tagen ist keine Beeinträchtigung der Transparenz des Copolyamids feststellbar.

Beispiel C

34 g 1-Isopropyl-10,10-dimethyl-1,11-diaminoundecan und 2 g 2,4,6-Tris-(dimethylaminomethyl)-phenol werden bei Raumtemperatur mit 100 g eines flüssigen, durch Kondensation von Bisphenol A mit Epichlorhydrin in Gegenwart von Alkali hergestellten Polyglycidyläthers mit einem Epoxidgehalt von 5,3 Epoxidäquivalenten/kg und einer Viskosität von 8000 – 12 000 m Pa · s bis 25°C gemischt.

Diese Mischung wird als Lack appliziert. An der härtbaren Mischung, die sich durch eine vorteilhaft lange Gebrauchsdauer auszeichnet, sowie am ausgehärteten Lack werden folgende Eigenschaften gemessen:

| | |
|---|---|
| Viskosität nach DIN*) 53 015 [m Pa · s] | = 600 |
| Gelierzeit (Tecan-Gerät) [Stunden] | = 11,15 |
| Staubtrockenzeit; Filmdicke 200 µm (Trockenzeitprüfgerät Typ 338 der Fa. Erichson [Stunden] | = 30 |
| Durchhärtungszeit (Trockenzeitprüfgerät wie oben) [Stunden] | = >30 |
| Härte nach Persoz; Filmdicke 200 µm; | |
| nach 1 Woche Lagerung bei 20°C [Sekunden] | = 350 |
| Erichsen-Test**) (DIN 53 156); Filmdicke 200 µm; | |
| nach 1 Woche Lagerung bei 20°C [mm] | = 3,0 |
| nach 1 Woche Lagerung bei 60°C [mm] | = 6,0 |
| Impact-Test; Filmdicke 200 µm; | |
| nach 1 Woche Lagerung bei 20°C [cm kg] | = 60 |
| nach 1 Woche Lagerung bei 60°C [cm kg] | = 90 |
| Dornbiege-Test (DIN 53 152); Filmdicke 200 µm; Dorn 15 mm | |
| nach 1 Woche Lagerung bei 20°C [Grad] | = 10 |
| nach 1 Woche Lagerung bei 60°C [Grad] | = 180 |

*) DIN = Deutsche Industrie-Norm

**) Die Prüfung wird bei 20°C/65% Luftfeuchtigkeit vorgenommen. Der Schlag erfolgt auf den Film, Gewicht des Prüfhammers: 1 kg; Durchmesser der aufschlagenden Halbkugel: 2 cm.

**Patentansprüche**

1. Eine Verbindung der Formel Ia

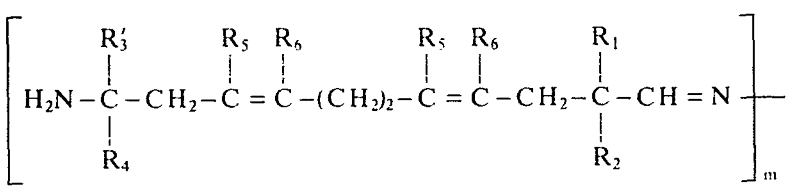

(Ia)

oder Ib

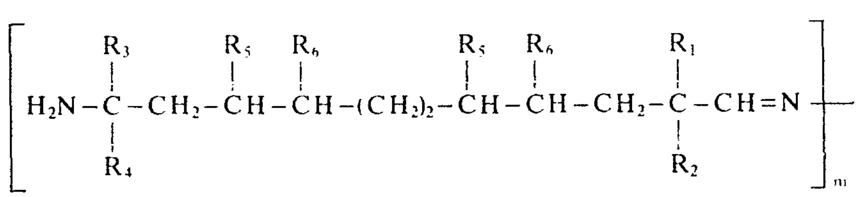

(Ib)

worin

m die Zahl 1 oder 2, Y, bei m = 1,

—OH, —$NH_2$, —NH—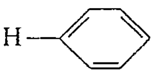, —$CH_2$—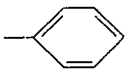 oder —$NHCONH_2$

und bei m = 2 die direkte Bindung,

$R_1$ Alkyl mit 1–12 C-Atomen,
$R_2$ Wasserstoff oder Alkyl mit 1–12 C-Atomen,
$R_3$ Alkyl mit 1–12 C-Atomen, Cycloalkyl mit 4–12 Ring-C-Atomen, Aralkyl mit 7 bis 8 C-Atomen, Aryl, das mit einer oder mehreren $C_1$–$C_4$-Alkylgruppen substituiert sein kann, Pyridyl, Furyl oder Thienyl,
$R_3'$ dasselbe wie $R_3$ oder, wenn $R_4$ Wasserstoff ist, auch —CH=CH—Alkyl oder —C(Alkyl)=CH—Alkyl mit je 1–4 C-Atomen in den Alkylteilen,
$R_4$ Wasserstoff, Alkyl mit 1–12 C-Atomen, Cycloalkyl mit 4–12 Ring-C-Atomen, Aralkyl mit 7 oder 8 C-Atomen oder gegebenenfalls mit einer oder mehreren $C_1$–$C_4$-Alkylgruppen substituiertes Aryl oder
$R_1$ und $R_2$ und/oder $R_3$ und $R_4$ bzw. $R_3'$ und $R_4$ zusammen Alkylen mit 3–11 C-Atomen und
$R_5$ und $R_6$ unabhängig voneinander Wasserstoff oder Methyl bedeuten.

2. Eine Verbindung der Formel Ia oder Ib nach Anspruch 1, worin $R_1$ Alkyl mit 1–5 C-Atomen, $R_2$ Wasserstoff oder Alkyl mit 1–5 C-Atomen oder $R_1$ und $R_2$ zusammen Alkylen mit 4–7 C-Atomen, $R_3$ Alkyl mit 1–7 C-Atomen, Cycloalkyl mit 5–8 C-Atomen oder unsubstituiertes Phenyl, $R_3'$ dasselbe wie $R_3$ oder bei $R_4$=H auch $C(C_2H_5)=CH-CH_3$, $R_4$ Wasserstoff oder Alkyl mit 1–5 C-Atomen und $R_5$ und $R_6$ je Wasserstoff bedeuten.

3. Eine Verbindung der Formel Ia oder Ib nach Anspruch 1 oder 2, worin m die Zahl 1, Y —OH, $R_1$ Alkyl mit 1–5 C-Atomen, $R_2$ Alkyl mit 1–5 C-Atomen oder Wasserstoff oder $R_1$ und $R_2$ zusammen Alkylen mit 4–7 C-Atomen, $R_3$ verzweigtes Alkyl mit 3–7 C-Atomen oder Cycloalkyl mit 5–8 C-Atomen, $R_3'$ dasselbe wie $R_3$ oder $-C(C_2H_5)=CH-CH_3$ und $R_4$, $R_5$ und $R_6$ je Wasserstoff bedeuten.

4. Eine Verbindung der Formel Ia oder Ib nach einem der Ansprüche 1–3, worin m die Zahl 1, Y = OH, $R_1$ und $R_2$ je Methyl oder Äthyl oder zusammen $C_{4-7}$-Alkylen, besonders Pentamethylen, $R_3$ oder $R_3'$ Isopropyl, 3-Pentyl, $C_{5-8}$-Cycloalkyl, besonders Cyclohexyl, und $R_4$, $R_5$ und $R_6$ je Wasserstoff bedeuten.

5. Ein Verfahren zur Herstellung einer Verbindung der Formel Ia oder Ib nach Anspruch 1, dadurch gekennzeichnet, daß man
A) eine Verbindung der Formel II

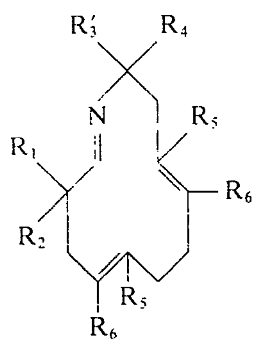

(II)

worin $R_1$ bis $R_6$ die unter Formel Ia bzw. Ib angegebenen Bedeutung haben, mit einer Verbindung der Formel IIIa oder IIIb

$$H_2N-Y \qquad \text{(IIIa)}$$

oder

$$[H_2NY \cdot H^{\oplus}]_n \quad X^{\ominus n} \qquad \text{(IIIb)}$$

worin X das Anion einer anorganischen, unter den Reaktionsbedingungen nichtoxidierenden Säure und n eine der Wertigkeit von X entsprechende ganze Zahl darstellen und Y die unter Formel Ia bzw. Ib angegebene Bedeutung hat, aber keine direkte Bindung darstellt, zu einer Verbindung der Formel Ia umsetzt, oder

B) eine Verbindung der Formel II katalytisch zu einer Verbindung der Formel IV

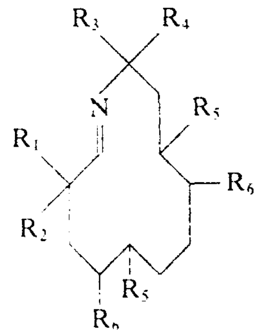

(IV)

hydriert, wobei $R_1$ bis $R_6$ die unter Formel Ia bzw. Ib angegebene Bedeutung haben, und die Verbindung der Formel IV mit einer Verbindung der Formel IIIa oder IIIb zu einer Verbindung der Formel Ib umsetzt.

## Claims

1. A compound of the formula Ia

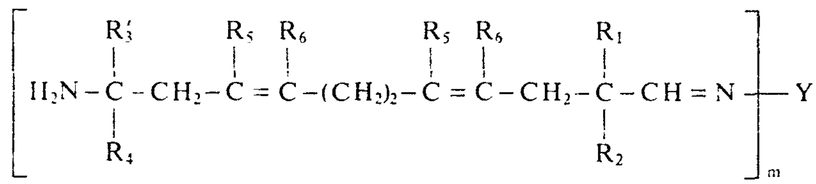

(Ia)

or Ib

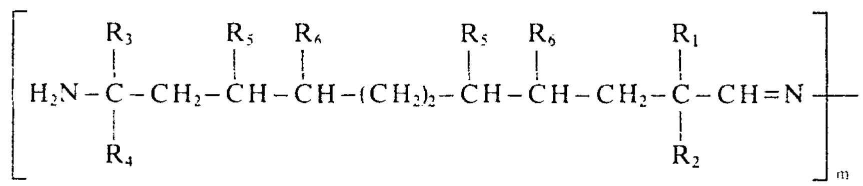

(Ib)

in which m is the number 1 or 2, Y is

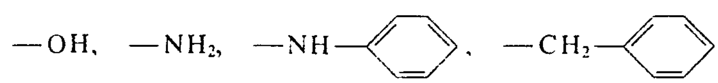

or $-NHCONH_2$ if m = 1 and is a direct bond if m = 2, $R_1$ is alkyl having 1 – 12 C atoms, $R_2$ is hydrogen or alkyl having 1 – 12 C atoms, $R_3$ is alkyl having 1 – 12 C atoms, cycloalkyl having 4 – 12 ring C atoms, aralkyl having 7 or 8 C atoms, aryl which can be substituted by one or more $C_1 - C_4$-alkyl groups, pyridyl, furyl or thienyl, $R_3'$ has the meaning defined for $R_3$ or, if $R_4$ is hydrogen, is also $-CH=CH-$alkyl or $-C(\text{alkyl})=CH-$alkyl, each having 1 – 4 C atoms in the alkyl moiety, and $R_4$ is hydrogen, alkyl having 1 – 12 C atoms, cycloalkyl having 4 – 12 ring C atoms, aralkyl having 7 or 8 C atoms or aryl which can be substituted by one or more $C_1 - C_4$-Alkyl groups, or $R_1$ and $R_2$ and/or $R_3$ and $R_4$, or $R_3'$ and $R_4$, together are alkylene having 3 – 11 C atoms, and $R_5$ and $R_6$ independently of one another are hydrogen or methyl.

2. A compound of the formula Ia or Ib according to claim 1, in which $R_1$ is alkyl having 1 – 5 C atoms and $R_2$ is hydrogen or alkyl having 1 – 5 C atoms, or $R_1$ and $R_2$ together are alkylene having 4 – 7 C atoms, $R_3$ is alkyl having 1 – 7 C atoms, cycloalkyl having 5 – 8 C atoms or unsubstituted phenyl, $R_3'$ has the meaning defined for $R_3$ or, if $R_4 = H$, is also $-C(C_2H_5)=CH-CH_3$, $R_4$ is hydrogen or alkyl having 1 – 5 C atoms and $R_5$ and $R_6$ are each hydrogen.

3. A compound of the formula Ia or Ib according to claim 1 or 2, in which m is the number 1, Y is $-OH$, $R_1$ is alkyl having 1 – 5 C atoms and $R_2$ is alkyl having 1 – 5 C atoms or hydrogen, or $R_1$ and $R_2$ together are alkylene having 4 – 7 C atoms, $R_3$ is branched alkyl having 3 – 7 C atoms or cycloalkyl having 5 – 8 C atoms, $R_3'$ has the meaning defined for $R_3$ or is $-C(C_2H_5)=CH-CH_3$ and $R_4$, $R_5$ and $R_6$ are each hydrogen.

4. A compound of the formula Ia or Ib according to any one of claims 1 to 3, in which m is the number 1, Y is —OH, $R_1$ and $R_2$ are each methyl or ethyl or together are $C_{4-7}$-alkylene, especially pentamethylene, $R_3$ or $R_3'$ are isopropyl, 3-pentyl or $C_{5-8}$-cycloalkyl, especially cyclohexyl, and $R_4$, $R_5$ and $R_6$ are each hydrogen.

5. A process for the preparation of a compound of the formula Ia or Ib according to claim 1, which comprises

A) reacting a compound of the formula II

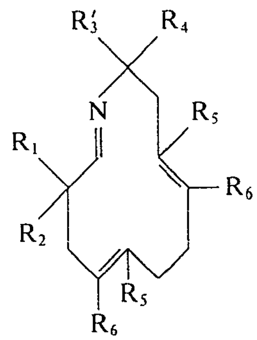

(II)

in which $R_1$ to $R_6$ are as defined under formula Ia or Ib, with a compound of the formula IIIa or IIIb

$$H_2N—Y \qquad \text{(IIIa)}$$

or

$$[H_2NY \cdot H^{\oplus}]_n \quad X^{\ominus n} \qquad \text{(IIIb)}$$

in which X is the anion of an inorganic acid which is nonoxidising under the reaction conditions and n is an integer corresponding to the valency of X and Y is as defined under formula Ia or Ib but is not a direct bond, to give a compound of the formula Ia, or

B) catalytically hydrogenating a compound of the formula II to a compound of the formula IV

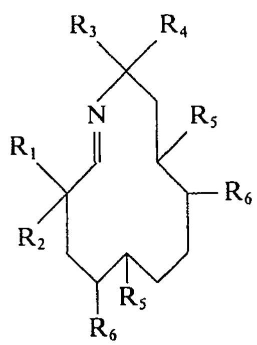

(IV)

in which $R_1$ to $R_6$ are as defined under formula Ia or Ib, and reacting the compound of the formula IV with a compound of the formula IIIa or IIIb to give a compound of the formula Ib.

**Revendications**

1. Composé répondant à l'une des formules Ia et Ib

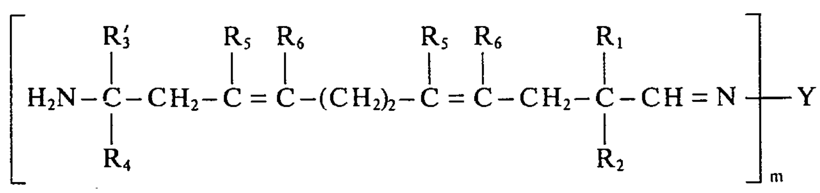

(Ia)

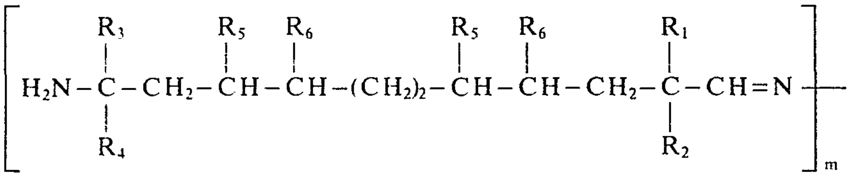 (Ib)

dans lesquelles m représente le nombre 1 ou le nombre 2, Y représente, dans le cas oú m est égal à 1, un radical

—OH, —$NH_2$, 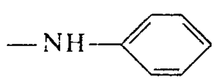, 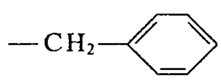

ou —$NHCONH_2$ et, lorsque m est égal à 2, une liaison directe.

$R_1$ représente un radical alkyle contenant de 1 à 12 atomes de carbone,
$R_2$ représente l'hydrogène ou un radical alkyle contenant de 1 à 12 atomes de carbone.
$R_3$ représente un radical alkyle contenant de 1 à 12 atomes de carbone, un radical cycloalkyle contenant de 4 à 12 atomes de carbone dans le cycle, un radical aralkyle à 7 ou 8 atomes de carbone, un radical aryle qui peut porter comme substituants un ou plusieurs radicaux alkyles en $C_1-C_4$, ou représente un radical pyridyle, furyle ou thiényle.
$R_3'$ a la même signification que $R_3$ ou, lorsque $R_4$ est l'hydrogène, peut également représenter un radical $-CH=CH-$Alkyl ou $-C($Alkyl$)=CH-$Alkyl contenant de 1 à 4 atomes de carbone dans chacune des parties alkyles.
$R_4$ représente l'hydrogène, un radical alkyle contenant de 1 à 12 atomes de carbone, un radical cycloalkyle contenant de 4 à 12 atomes de carbone dans le cycle, un radical alkyle à 7 ou 8 atomes de carbone ou un radical aryle éventuellement porteur d'un ou plusieurs radicaux alkyles en $C_1-C_4$,

ou les couples ($R_1$, $R_2$) et/ou ($R_3$, $R_4$) ou ($R_3'$, $R_4$) forment chacun un radical alkylène contenant de 3 à 11 atomes de carbone, et,

$R_5$ et $R_6$ représentent chacun, indépendamment l'un de l'autre, l'hydrogène ou un radical méthyle.

2. Composé de formule Ia ou Ib selon la revendication 1, caractérisé en ce que $R_1$ représente un alkyle en $C_1-C_5$, $R_2$ est l'hydrogène ou un alkyle en $C_1-C_5$, ou encore $R_1$ et $R_2$ forment ensemble un alkylène en $C_4-C_7$, $R_3$ représente un alkyle en $C_1-C_7$, un cycloalkyle en $C_5-C_8$ ou un phényle non substitué, $R_3'$ a la même signification que $R_3$ ou, dans le cas ou $R_4$ est H, peut également représenter un radical $-C(C_2H_5)=CH-CH_3$, $R_4$ représente l'hydrogène ou un alkyle en $C_1-C_5$ et $R_5$ et $R_6$ représente chacun l'hydrogène.

3. Composé de formule Ia ou Ib selon l'une des revendications 1 et 2, caractérisé en ce que m est égal à 1, Y représente $-OH$, $R_1$ représente un alkyle en $C_1-C_5$, $R_2$ un alkyle en $C_1-C_5$ ou l'hydrogène, ou encore $R_1$ et $R_2$ forment ensemble un alkylène en $C_4-C_7$, $R_3$ représente un alkyle ramifié contenant de 3 à 7 atomes de carbone, ou un cycloalkyle en $C_5-C_8$, $R_3'$ a la même signification que $R_3$ ou représente un radical $-C(C_2H_5)=CH-CH_3$ et $R_4$, $R_5$ et $R_6$ représentent chacun l'hydrogène.

4. Composé de formule Ia ou Ib selon l'une quelconque des revendications 1 à 3, caractérisé en ce que m désigne le nombre 1, Y représente $-OH$, $R_1$ et $R_2$ représentent chacun un radical méthyle ou éthyle ou forme ensemble un radical alkylène en $C_4-C_7$, en particulier un radical pentaméthylène, $R_3$ ou $R_3'$ représente un isopropyle, un pentyle-3 ou un cycloalkyle en $C_5-C_8$, en particulier un cyclohexyle, et $R_4$, $R_5$ et $R_6$ représentent chacun l'hydrogène.

5. Procédé de préparation d'un composé de formule Ia ou Ib selon la revendication 1, procédé caractérisé en ce que:

A) On fait réagir un composé répondant à la formule II

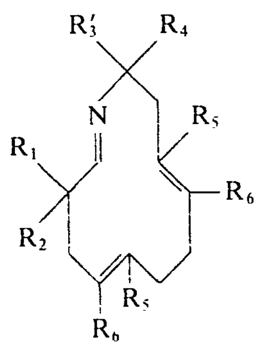 (II)

dans laquelle $R_1$ à $R_6$ ont les significations qui ont été données sous les formules Ia et Ib, avec un composé répondant à l'une des formules IIIa et IIIb

$$H_2N — Y \quad \text{(IIIa)}$$

$$[H_2NY \cdot H^{\oplus}]_n \quad X^{\ominus n} \quad \text{(IIIb)}$$

où X représente l'anion d'un acide minéral n'ayant pas de propriétés oxydantes dans les conditions de la réaction, n est un nombre entier correspondant à la valence de X et Y a la signification qui a été donnée sous les formules Ia et Ib mais ne représente pas une liaison directe, de manière à obtenir un composé de formule Ia, ou,

B) on hydrogène catalytiquement un composé de formule II afin de le convertir en un composé répondant à la formule IV

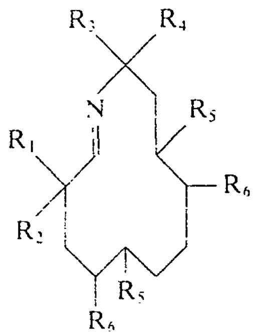

(IV)

dans laquelle $R_1$ à $R_6$ ont les significations qui ont été données sous les formules Ia et Ib, et on fait réagir le composé de formule IV avec un composé de formule IIIa ou IIIb de manière à obtenir un composé de formule Ib.